# EUROPÄISCHE PATENTSCHRIFT

(11) **EP 3 253 848 B1**
(45) Veröffentlichungstag und Bekanntmachung des Hinweises auf die Patenterteilung: **25.12.2019**
(21) Anmeldenummer: 16703950.2
(22) Anmeldetag: 04.02.2016
(51) Int. Cl.: C09K 11/06, C07D 215/06, C07D 519/00, C07D 209/86, C07D 219/02, C07D 279/22, C07D 279/16, C07D 209/08, C07D 265/38, C07D 487/04, C07D 265/36, C07D 235/18, C07D 223/22, C07D 401/14, H01L 51/50

(54) **BLAUE FLUORESZENZEMITTER**
BLUE FLUORESCENT EMITTERS
ÉMETTEURS FLUORESCENTS BLEUS

(30) Priorität: 06.02.2015 DE 102015101767
(43) Veröffentlichungstag der Anmeldung: 13.12.2017
(73) Patentinhaber: Technische Universität Dresden, 01069 Dresden (DE)
(72) Erfinder: LYGAITIS, Ramunas, 01326 Dresden (DE); SCHOLZ, Reinhard, 01705 Freital (DE); ZEIKA, Olaf, 01662 Meissen (DE); REINEKE, Sebastian, 01099 Dresden (DE); LEO, Karl, 01219 Dresden (DE); HOFMANN, Simone, 01159 Dresden (DE); OBERLÄNDER, Martin, 01099 Dresden (DE)
(74) Vertreter: Viering, Jentschura & Partner mbB Patent- und Rechtsanwälte
(86) Internationale Anmeldenummer: PCT/EP2016/052413
(87) Internationale Veröffentlichungsnummer: WO 2016/124704

(56) Entgegenhaltungen:
- EP-A2- 0 455 247
- WO-A1-2012/121561
- WO-A1-2015/009076
- WO-A1-2015/195837
- JP-A- 2005 060 382
- JP-A- 2011 162 490
- US-A1- 2006 051 616
- US-A1- 2007 099 025
- US-A1- 2014 131 686
- US-A1- 2014 225 070
- US-A1- 2014 326 961
- VARGOVA ET AL: "Intermolecular and intramolecular coupling in charged monosubstituted hexapyrrolylbenzenes", ELECTROCHIMICA ACTA, ELSEVIER SCIENCE PUBLISHERS, BARKING, GB, Bd. 52, Nr. 28, 25. August 2007 (2007-08-25), Seiten 7885-7894, XP022214043, ISSN: 0013-4686, DOI: 10.1016/J.ELECTACTA.2007.06.049
- CHRISTOPHER J A ET AL: "Synthetic utility of 4-bromo-2,3,5,6-tetrafluoropyridine", JOURNAL OF FLUORINE CHEMISTRY, ELSEVIER, NL, Bd. 129, Nr. 5, 1. Mai 2008 (2008-05-01), Seiten 447-454, XP022615601, ISSN: 0022-1139, DOI: 10.1016/J.JFLUCHEM.2008.01.004 [gefunden am 2008-01-19]
- DONGDONG ZHAO ET AL: "Synthesis, crystal structures, and photophysical properties of a series of novel tetrahydrobenzodiacridines", JOURNAL OF LUMINESCENCE, vol. 134, 1 February 2013 (2013-02-01), pages 566-575, XP055475333, NL ISSN: 0022-2313, DOI: 10.1016/j.jlumin.2012.07.028
- OH YOUNG KIM ET AL: "High efficiency thermally activated delayed fluorescent devices using a mixed host of carbazole and phosphine oxide derived host materials", SYNTHETIC METALS, vol. 201, 23 January 2015 (2015-01-23), pages 49-53, XP055475336, CH ISSN: 0379-6779, DOI: 10.1016/j.synthmet.2014.12.025

## Beschreibung

Gegenstand der vorliegenden Erfindung sind Verbindungen der Formeln (II), (III) und (IV) wie hierin definiert, sowie deren Verwendung als Emitter oder Trägermaterial in einem optoelektronischen Bauelement.

Die Entwicklung von neuartigen funktionellen Verbindungen zur Verwendung in elektronischen Vorrichtungen ist aktuell Gegenstand intensiver Forschung. Ziel ist hierbei die Entwicklung und Untersuchung von Verbindungen, welche bislang noch nicht in elektronischen Vorrichtungen eingesetzt wurden, sowie die Entwicklung von Verbindungen, welche ein verbessertes Eigenschaftsprofil der Vorrichtungen ermöglichen.

Unter dem Begriff optoelektronisches Bauelement werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Field-Quench-Devices (OFQDs), organische lichtemittierende elektrochemische Zellen (OLECs), organische Laserdioden (O-Laser) und organische Elektrolumineszenzvorrichtungen (OLEDs) verstanden.

Der Aufbau organischer Elektrolumineszenzvorrichtungen (OLEDs), in denen die hierin beschriebenen Verbindungen bevorzugt als funktionelle Materialien eingesetzt werden können, ist dem Fachmann bekannt und wird unter anderem in den Patentveröffentlichungen US 4539507, US 5151629, EP 0676461 und WO 1998/27136 beschrieben.

Betreffend die Leistungsdaten der OLEDs sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich sublimieren lassen, ohne sich dabei zu zersetzen. Betreffend die Leistungsdaten der OLEDs sind, insbesondere in Hinblick auf eine breite kommerzielle Verwendung, noch weitere Verbesserungen erforderlich. Von besonderer Bedeutung sind in diesem Zusammenhang die Lebensdauer, die Effizienz und die Betriebsspannung der OLEDs sowie die realisierten Farbwerte und der Farbwiedergabeindex.

Insbesondere werden in der Industrie dringend langlebige, effiziente blaue Emitter für OLEDs, die obendrein auch noch kostengünstig herstellbar sind, benötigt.

Die im Zuge der Rekombination von Löchern und Elektronen in der Emitterschicht gebildeten Exzitonen teilen sich im Verhältnis 1:3 auf Singulett- und Triplett-Zustände auf. Die Verwendung von organisch-metallischen Komplexstrukturen zieht Nutzen aus den ansonsten über nicht-radiative Löschprozesse für die Elektrolumineszenz verlorene Triplett-Exzitonen. Insbesondere mit Iridium oder anderen Edelmetallen dotierte Matrixmaterialien, die gemäß dem Stand der Technik häufig Carbazolderivate, zum Beispiel Bis(carbazolyl)biphenyl, weiterhin Ketone (WO 2004/093207), Phosphinoxide, Sulfone (WO 2005/003253), Triazinverbindungen wie Triazinylspirobifluoren (WO 2005/053055 und WO 2010/05306) enthalten, finden Anwendung als phosphoreszierende Emitter, darüber hinaus werden aber auch Metallkomplexe, beispielsweise Bis(2-methyl-8-quinolinolato-N1,O8)-(1,1'-Biphenyl-4-olato)aluminum (BAIq) oder Bis[2-(2-benzothiazol)phenolat]-zink(II) verwendet.

Aufgrund von Spinrestriktionen im Rekombinationsprozess der Elektronen und Löcher in der Emitterschicht können in fluoreszierenden Emittern folglich nur maximal 25 % der elektrischen Energie in Licht umgewandelt werden. In Materialien, in denen der Triplett-Zustand energetisch hoch und somit nahe am Singulett-Zustand gelegen ist, ist *reverse intersystem crossing* (RISC) möglich. Dabei werden Triplett-Exzitonen thermisch in den Singulett-Zustand angehoben, was als *singulet harvesting* bezeichnet wird. So kann theoretisch bis zu 100 % der Energie, die in den energetisch angeregten Zuständen gespeichert ist, in Form von fluoreszenter Elektrolumineszenz abgestrahlt werden. Organische Moleküle, die sich aus Donor- und Akzeptor-Strukturen zusammensetzen, deren höchstes besetztes Molekülorbital (HOMO) und niedrigstes unbesetztes Molekülorbital (LUMO) geringfügig überlappen, stellen effiziente und kostengünstige Alternativen zu Edelmetall-dotierten organischen Matrixmaterialien dar. Insbesondere könnten in diesem Zusammenhang mit p-elektronenreichen, Stickstoff-haltigen Heterozyklen substituierte p-elektronenarme Zyklen durch ihre intensive, thermisch aktivierte verzögerte Fluoreszenz Abhilfe schaffen.

Ferner offenbart die WO 2015/009076 A1 eine spezifische Kombination einer Dotiermittelverbindung, die ein Aminopyren umfasst, und einer Wirtsverbindung, die ein Diaryl-substituiertes Anthracen umfasst, und einer organischen Elektrolumineszenzvorrichtung, welche die Dotiermittelverbindung umfasst.

Die US 2014/326961 A1 beschreibt spezifische Verbindungen auf Benzofluoren-Basis sowie eine OLED, die diese Verbindung umfasst.

Die US 2014/225070 A1 offenbart eine spezifische Verbindung, welche eine Naphtylanthracengruppe und eine Arylaminegruppe umfasst und eine OLED, die diese Verbindung umfasst.

In der US 2014/131686 A1 ist eine Verbindung, die so konfiguriert ist, dass sie einen ersten Peak bei einem Masse / Ladungsverhältnis (m/z) von 773, einen zweiten Peak bei einem Masse / Ladungsverhältnis (m/z) von 349 und einen dritten Peak bei einem Masse / Ladungsverhältnis (m/z) von 425 bei Analyse der Verbindung durch Massenspektrometrie ergibt, beschrieben.

US 2007/099025 A1 offenbart ein organisches elektrolumineszentes Element, umfassend eine Anode und eine Kathode, zwischen denen eine Emissionsschicht, die eine phosphoreszierende Verbindung enthält, und eine an die Emissionsschicht angrenzende Schicht, wobei die an die Emissionsschicht angrenzende Schicht zwischen der Emissionsschicht und der Kathode angeordnet ist, wobei die Schicht, die zur Emissionsschicht benachbart ist eine Verbindung mit einer Elektronen ziehenden Gruppe umfasst; und ein HOMO-Niveau von -5,7 bis -7,0 eV und ein LUMO-Niveau von -1,3 bis -2,3 eV aufweist.

Die US 2006/051616 A1 beschreibt eine organische Verbindung, bestehend aus elementarem Wasserstoff und mindestens zwei Arten von Elementen, ausgewählt aus der Gruppe bestehend aus Elementen, die zur dreizehnten Gruppe bis zur siebzehnten Gruppe der zweiten Periode bis zur fünften Periode des Periodensystems gehören, und die gleichzeitig Fluoreszenz emittieren können und Phosphoreszenz bei einer Temperatur von -30 °C oder mehr und 100 °C oder weniger aufweisen.

Schließlich offenbart die JP 2005 060382 A spezifische organische aromatische Verbindungen und eine OLED, welche die Verbindungen enthält.

Im Rahmen der vorliegenden Erfindung wurde nun überraschenderweise gefunden, dass die Verbindungen der Formeln (II), (III) und (IV) hervorragend zur Verwendung in optoelektronischen Bauelementen, insbesondere als Emitter oder Matrixmaterial, geeignet sind. Die Verbindungen der Formeln (II), (III) und (IV) zeichnen sich durch ihre energetisch hoch liegenden Triplett-Zustände aus, wodurch der tiefste angeregte Singulett-Zustand der Verbindungen der Formeln (II), (III) und (IV) thermisch über diesem Triplett-Zustand besetzt werden kann, so dass die Effizienz bei der Energieumwandlung theoretisch Werte von bis zu 100 % erreichen kann.

Die ersten, zweiten und dritten Gegenstände der vorliegenden Erfindung betreffen spezifische Verbindungen der Formeln (II), (III) und (IV) wie in den angefügten unabhängigen Ansprüchen 1 bis 3 definiert.

Ein weiterer Gegenstand der vorliegenden Erfindung ist darüber hinaus die Verwendung mindestens einer der hierin beschriebenen Verbindungen in einem optoelektronischen Bauelement, beispielsweise einer elektronischen Elektrolumineszenzvorrichtung (OLED), einer organischen integrierten Schaltung (O-IC), einem organischen Feld-Effekt-Transistor (O-FET), einem organischen Dünnfilmtransistor (O-TFT), einem organischen lichtemittierenden Transistor (O-LET), einer organischen Solarzelle (O-SC), einem organischen optischen Detektor, einem organischen Photorezeptor, einer organischen Feld-Quench-Vorrichtung (O-FQD), einer lichtemittierenden elektrochemischen Zelle (LEC) oder einer organischen Laserdiode (O-Laser).

Schließlich betrifft die Erfindung auch optoelektronische Bauelemente, wie die vorstehend genannten, die mindestens eine der hierin beschriebenen Verbindungen enthalten.

"Mindestens ein", wie hierin verwendet, bedeutet 1 oder mehr, d.h. 1, 2, 3, 4, 5, 6, 7, 8, 9 oder mehr. "Mindestens ein Substituent" bedeutet somit beispielsweise mindestens eine Art von Substituent, d.h. dass eine Art von Substituent oder eine Mischung mehrerer verschiedener Substituenten gemeint sein kann.

Die erfindungsgemäßen Verbindungen sind Verbindungen der allgemeinen Strukturformeln (II), (III) und (IV) wie in den angefügten unabhängigen Ansprüchen 1 bis 3 definiert.

Sofern hierin nicht anders definiert, enthält eine Arylgruppe im Sinne dieser Erfindung 6 bis 60 aromatische Ringatome und eine Heteroarylgruppe im Sinne dieser Erfindung 5 bis 60 aromatische Ringatome, von denen mindestens eines ein Heteroatom darstellt.

Weiterhin wird unter einer Arylgruppe bzw. Heteroarylgruppe im Rahmen der vorliegenden Erfindung entweder eine monozyklische aromatische Gruppe, wie z.B. Phenyl, bzw. eine monozyklische heteroaromatische Gruppe, wie beispielsweise Pyridinyl, Pyrimidinyl oder Thienyl, oder eine kondensierte (annellierte, mehrkernige) aromatische bzw. heteroaromatische polyzyklische Gruppe, beispielsweise Naphthalinyl, Phenanthrenyl oder Carbazolyl verstanden. Ein kondensierter (annellierter, mehrkerniger) aromatischer bzw. heteroaromatischer Polyzyklus besteht im Sinne der vorliegenden Anmeldung aus zwei oder mehr miteinander kondensierten einfachen (einkernigen) aromatischen bzw. heteroaromatischen Ringen.

Ein Stickstoff-haltiges Heteroaryl (HetAr) bezeichnet im Sinne der vorliegenden Erfindung ein aromatisches Ringsystem, welches mindestens ein Stickstoffatom sowie optional weitere Heteroatome, bevorzugt ausgewählt aus S und O, enthält. Das Stickstoff-haltige Heteroaryl ist als elektronenreicher Substituent D über das mindestens eine Stickstoffatom an den Aromaten C der allgemeinen Strukturformeln (II), (III) oder (IV) angebunden. Die Stickstoff-haltigen Heteroarylreste können substituiert sein, wobei die Substituenten beispielsweise ausgewählt werden aus Halogenen, substituierten oder unsubstituierten geradkettigen Alkyl-, Alkoxy- oder Thioalkylgruppen mit 1 bis 20 C-Atomen oder substituierten oder unsubstituierten, verzweigten oder cyclischen Alkyl-, Alkoxy- oder Thioalkylgruppen mit 3 bis 20 C-Atomen oder substituierten oder unsubstituierten Alkenyl- oder Alkinylgruppen mit 2 bis 20 C-Atomen, wobei jede der vorgenannten Gruppen ein oder mehrere Heteroatome ausgewählt aus Si, O, S, Se, N und P beinhalten kann. Bevorzugte Substituenten der Stickstoff-haltigen Heteroarylreste sind substituierte oder unsubstituierte Methyl-, Ethyl-, Propyl-, Isopropyl-, c-Propyl-, n-Butyl-, sec-Butyl-, Isobutyl- und t-Butylgruppen und ihre korrespondierenden Alkoxy- und Thioalkyl-Äquivalente. Für den Fall, dass die vorgenannten Substituenten der Stickstoff-haltigen Heteroarylreste wiederum substituiert sind, werden deren Substituenten ausgewählt aus geradkettigen Alkyl-, Alkoxy- oder Thialkylgruppen mit 1 bis 20 C-Atomen, cyclischen oder verzweigten Alkyl-, Alkoxy- oder Thioalkylgruppen mit 3 bis 20 C-Atomen, Alkenyl- oder Alkinylgruppen mit 2 bis 20 C-Atomen, Carboxyl-, Hydroxyl-, Thiol-, Amino-, Hydrazin- oder Nitrogruppen, Aryl- oder Heteroarylgruppen wie nachstehend definiert, sowie Halogenen, insbesondere Fluor, und Pseudohalogenen (-CN, -N₃, -OCN, -NCO, -CNO, -SCN, -NCS, -SeCN).

Im Rahmen der vorliegenden Erfindung werden unter einem Stickstoff-haltigen Heteroaryl, welches mit weiteren Resten substituiert sein kann, insbesondere Gruppen verstanden, welche ausgewählt sind aus bzw. abgeleitet sind von Pyrrolyl, Indolyl, Isoindolyl, Carbazolyl, Pyridinyl, Chinolinyl, Isochinolinyl, Acridinyl, Phenanthridinyl, Benzo-5,6-chinolinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, Oxazolyl, Benzoxazolyl, Naphthoxazolyl, Anthroxazolyl, Phenanthroxazolyl, Isoxazolyl, 1,2-Thiazolyl, 1,3-Thiazolyl, Benzothiazolyl, Pyridazinyl, Benzopyridazinyl, Pyrimidinyl, Benzpyrimidinyl, Chinoxalinyl, Pyrazinyl, Phenazinyl, Naphthyridinyl, Carbazolyl, Benzocarbolinyl, Phenanthrolinyl, Purinyl, Pteridinyl uns Indolizinyl.

In Sinne der vorliegenden Erfindung bezeichnet die Formel -N(Ar)₂ ein Diarylamin, welches über das Stickstoffatom an den Aromaten C der allgemeinen Formeln (II), (III) oder (IV) gebunden ist, und welches zwei aromatische Substituenten trägt (Ar). Die beiden Aryl-Substituenten können dabei identisch oder verschieden sein. In verschiedenen Ausführungsformen werden sie ausgewählt aus den unten genannten aromatischen Gruppen. Im Gegensatz zu Heteroarylresten beinhalten die beiden aromatischen Substituenten Ar des Diarylaminrestes -N(Ar)₂ keine Heteroatome in ihren aromatischen Ringstrukturen. Sie können allerdings substituiert sein, wobei die Substituenten vorzugsweise aus den oben im Zusammenhang mit den Stickstoff-haltigen Heteroarylresten beschriebenen Substituenten ausgewählt werden.

Im Rahmen der vorliegenden Erfindung werden unter einer Aryl(Ar)-Gruppe, die jeweils mit weiteren Resten substituiert sein kann, insbesondere Gruppen verstanden, welche ausgewählt werden aus der Gruppe bestehend aus Phenyl, Naphthyl, Anthracenyl, Phenanthrenyl, Fluorenyl, Pyrenyl, Dihydropyrenyl, Chrysenyl, Perylenyl, Fluoranthenyl, Benzanthracenyl, Benzphenanthrenyl, Tetracenyl, Pentacenyl und Benzpyrenyl, wobei die vorstehend genannten Gruppen jeweils substituiert oder unsubstituiert sein können. Wenn sie substituiert sind, werden die Substituenten vorzugsweise aus den oben im Zusammenhang mit den Stickstoff-haltigen Heteroarylresten beschriebenen Substituenten ausgewählt. Darüber hinaus können Arylgruppen im Sinne der vorliegenden Erfindung auch über ihre Substituenten miteinander verbrückt sein. Beispiele entsprechender verbrückter Diarylamine sind Iminodibenzyle (10,11-Dihydrobenzazepine) oder 9H-Acridin, aber auch Phenoxazine und Phenothiazine können so verstanden werden.

Die Formel -N(HetAr)(Ar)bezeichnet im Sinne der vorliegenden Erfindung ein Amin, welches mit einer Aryl- und einer Heteroarylgruppe substituiert ist, und über das Stickstoffatom an den Aromaten C der allgemeinen Formel (I) gebunden ist. Die Arylgruppe ist wie vorstehend definiert. Die Heteroarylgruppe bezeichnet ein aromatisches Ringsystem, welches mindestens ein Heteroatom, bevorzugt ausgewählt aus N, S und O, beinhaltet, und welches darüber hinaus substituiert sein kann, wobei die Substituenten vorzugsweise aus den oben im Zusammenhang mit den Stickstoff-haltigen Heteroarylresten beschriebenen Substituenten ausgewählt werden.

Analog bezeichnet die Formel -N(HetAr)₂ ein Amin, welches zwei Heteroaryl-Substituenten trägt und welches über das Stickstoffatom an den Aromaten C der allgemeinen Strukturformel (I) gebunden ist. Die Heteroarylgruppen können identisch oder verschieden sein und sind wie vorstehend definiert. Insbesondere können sie auch substituiert sein, wobei die Substituenten vorzugsweise aus den oben im Zusammenhang mit den Stickstoff-haltigen Heteroarylresten beschriebenen Substituenten ausgewählt werden.

Im Rahmen der vorliegenden Erfindung wird unter einer Heteroaryl(HetAr)-Gruppe, die jeweils mit weiteren Resten substituiert und über eine beliebige Position am Aromaten verknüpft sein kann, insbesondere eine Gruppe verstanden, welche ausgewählt wird aus der Gruppe bestehend aus Furanyl, Difuranyl, Terfuranyl, Benzofuranyl, Isobenzofuranyl, Dibenzofuranyl, Thienyl, Dithienyl, Terthienyl, Benzothienyl, Isobenzothienyl, Benzodithienyl, Benzotrithienyl, Pyrrolyl, Indolyl, Isoindolyl, Carbazolyl, Pyridinyl, Chinolinyl, Isochinolinyl, Acridinyl, Phenanthridinyl, Benzo-5,6-chinolinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, Oxazolyl, Benzoxazolyl, Naphthoxazolyl, Anthroxazolyl, Phenanthroxazolyl, Isoxazolyl, 1,2-Thiazolyl, 1,3-Thiazolyl, Benzothiazolyl, Pyridazinyl, Benzopyridazinyl, Pyrimidinyl, Benzpyrimidinyl, Chinoxalinyl, Pyrazinyl, Phenazinyl, Naphthyridinyl, Carbazolyl, Benzocarbolinyl, Phenanthrolinyl, Phenoxazine, Phenothiazine, Iminostilbene, Purinyl, Pteridinyl und Indolizinyl sowie annelierte Systeme der vorgenannten untereinander und/oder mit Arylgruppen, wie beispielsweise Naphthyl, Anthracenyl, Phenanthrenyl, Fluorenyl, Pyrenyl, Dihydropyrenyl, Chrysenyl, Perylenyl, Fluoranthenyl, Benzanthracenyl, Benzphenanthrenyl, Tetracenyl, Pentacenyl und Benzpyrenyl, wobei die vorstehend genannten Gruppen jeweils substituiert oder unsubstituiert sein können. Wenn sie substituiert sind, werden die Substituenten vorzugsweise aus den oben im Zusammenhang mit den Stickstoff-haltigen Heteroarylresten beschriebenen Substituenten ausgewählt. Darüber hinaus können Heteroarylgruppen im Sinne der vorliegenden Erfindung auch über ihre Substituenten miteinander verbrückt sein.

Verbindungen gemäß einer der Formeln (Ia) bis (VIc), die nicht erfindungsgemäß sind, sind offenbart.

Insbesondere ist eine Verbindung der allgemeinen Formel (Ia) offenbart: 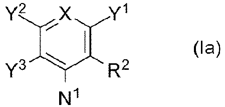 wobei
X C-CF₃, C-Cl, C-F, C-CN oder N ist;
R² F, Cl, Br, C1-C12- Perfluoralkyl, insbesondere CF₃, CN oder H ist;
N¹ ein Stickstoff-haltiger Heteroarylrest, der über ein Stickstoffatom gebunden ist, oder ein Rest der Formel -N(Ar)₂, -N(HetAr)(Ar), -N(HetAr)₂ ist, wobei jedes Ar unabhängig ein Arylrest und jedes HetAr unabhängig ein Heteroarylrest ist;
Y¹, Y² und Y³ jeweils unabhängig voneinander C1-C12 Perfluoralkyl, insbesondere CF₃, CCl₃, F, Cl, Br, SCN, CN, H, ein Stickstoff-haltiger Heteroarylrest, der über ein Stickstoffatom gebunden ist, oder ein Rest der Formel -N(Ar)₂, -N(HetAr)(Ar), -N(HetAr)₂ ist, wobei jedes Ar unabhängig ein Arylrest und jedes HetAr unabhängig ein Heteroarylrest ist;
mit der Maßgabe, dass,
a) nur einer der Substituenten Y¹⁻³ und R² CN ist;
b) wenn X C-CN ist, keiner der Substituenten Y¹⁻³ und R² CN ist.
c) wenn X C-F, C-Cl oder C-CN ist, und Y¹ und Y² jeweils unabhängig voneinander ein Stickstoff-haltiger Heteroarylrest, der über ein Stickstoffatom gebunden ist, oder ein Rest der Formel - N(Ar)₂, -N(HetAr)(Ar), -N(HetAr)₂, wobei jedes Ar unabhängig ein Arylrest und jedes HetAr unabhängig ein Heteroarylrest ist, sind, Y³ nicht F, Cl, Br oder CN ist.

In verschiedenen Ausführungsformen sind die Verbindungen der Formel (la) auf solche Verbindungen beschränkt, in denen, wenn X C-CN ist, N¹ ein über das Stickstoffatom gebundenes Carbazol ist und Y¹, Y² und Y³ jeweils ein über das Stickstoffatom gebundenes Carbazol sind, R² nicht F ist.

In verschiedenen Ausführungsformen der Verbindungen der Formel (la) handelt es sich bei dem Stickstoff-haltigen Heteroarylrest (N¹) um Pyrrolyl, Indolyl, Isoindolyl, Carbazolyl, Piperidinyl, Tetrahydrochinolinyl, Pyrrolidinyl, Tetrahydroisochinolinyl, Acridinyl, Phenanthridinyl, Benzo-5,6-chinolinyl, Benzomorpholinyl, Benzothiomorpholinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, , Benzopyridazinyl, , Benzpyrimidinyl, Chinoxalinyl, Pyrazinyl, Phenazinyl, Naphthyridinyl, Carbazolyl, Benzocarbolinyl, , Purinyl, Pteridinyl, oder Indolizinyl. Besonders bevorzugt sind dabei Indolyl, Isoindolyl, Carbazolyl, Pyridinyl, Chinolinyl, Isochinolyinyl, Acridinyl und Phenanthridinyl. Die vorgenannten können substituiert oder unsubstituiert sein, wobei es sich bei den Substituenten der vorgenannten Stickstoff-haltigen Heteroarylreste beispielsweise um Substituenten ausgewählt aus der Gruppe bestehend aus Wasserstoff, Halogenen, substituierten oder unsubstituierten geradkettigen Alkyl-, Alkoxy- oder Thioalkylgruppen mit 1 bis 20 C-Atomen oder substituierten oder unsubstituierten, verzweigten oder cyclischen Alkyl-, Alkoxy- oder Thioalkylgruppen mit 3 bis 20 C-Atomen oder substituierten oder unsubstituierten Alkenyl- oder Alkinylgruppen mit 2 bis 20 C-Atomen handelt, wobei jede der vorgenannten Gruppen ein oder mehrere Heteroatome ausgewählt aus Si, O, S, Se, N und P beinhalten kann. Gesetz den Fall, dass die vorgenannten Substituenten der Stickstoff-haltigen Heteroarylreste wiederum substituiert sind, sind deren Substituenten ausgewählt aus geradkettigen Alkyl-, Alkoxy- oder Thialkylgruppen mit 1 bis 20 C-Atomen, cyclischen oder verzweigten Alkyl-, Alkoxy- oder Thioalkylgruppen mit 3 bis 20 C-Atomen, Alkenyl- oder Alkinylgruppen mit 2 bis 20 C-Atomen, Hydroxyl-, Thiol-, Amino-, Hydrazin-, Nitro- oder Nitrilgruppen, unsubstituierten Aryl- oder Heteroarylgruppen wie bereits defininert, sowie Halogenen. Bevorzugte Substituenten der Stickstoff-haltigen Heteroarylreste sind Wasserstoff, Methyl-, Ethyl-, Propyl-, Isopropyl-, c-Propyl-, n-Butyl-, sec-Butyl-, Isobutyl- und t-Butylgruppen und ihre korrespondierenden Alkoxy- und Thioalkyl-Äquivalente. Die vorgenannten Stickstoff-haltigen Heteroarylreste können darüber hinaus mit einem oder mehreren Arylrest(en) oder mit einem oder mehreren Heteroarylrest(en) anneliert sein. Bevorzugte Beispiele eines solchen annelierten Systems sind Dibenzazepin und 2,3-Indolocarbazol. Auch solche annelierten Aryl(Heteroaryl)-Systeme können wiederum substituiert oder unsubstituiert sein. Bevorzugte Substituenten sind dabei wie voranstehend definiert. Ein bevorzugtes Beispiel eines solchen substituierten, annelierten Systems ist 2,3-(1-Methylindolo)carbazol.

In verschiedenen Ausführungsformen der Verbindungen der Formel (la) handelt es sich bei den Arylresten in den Resten der Formeln -N(Ar)₂ und -N(HetAr)(Ar) unabhängig voneinander bevorzugt um substituiertes oder unsubstituiertes Phenyl, Naphthyl, Anthracenyl, Phenanthrenyl, Fluorenyl, Pyrenyl, Dihydropyrenyl, Chrysenyl, Perylenyl, Fluoranthenyl, Benzanthracenyl, Benzphenanthrenyl, Tetracenyl, Pentacenyl und Benzpyrenyl. Bei den Substituenten der vorgenannten Arylreste handelt es sich beispielsweise um Substituenten ausgewählt aus der Gruppe bestehend aus Halogenen, substituierten oder unsubstituierten geradkettigen Alkyl-, Alkoxy- oder Thioalkylgruppen mit 1 bis 20 C-Atomen oder substituierten oder unsubstituierten, verzweigten oder cyclischen Alkyl-, Alkoxy- oder Thioalkylgruppen mit 3 bis 20 C-Atomen oder substituierten oder unsubstituierten Alkenyl- oder Alkinylgruppen mit 2 bis 20 C-Atomen handelt, wobei jede der vorgenannten Gruppen ein oder mehrere Heteroatome ausgewählt aus Si, O, S, Se, N und P beinhalten kann. Gesetz den Fall, dass die vorgenannten Substituenten der Arylreste wiederum substituiert sind, sind deren Substituenten ausgewählt aus geradkettigen Alkyl-, Alkoxy- oder Thialkylgruppen mit 1 bis 20 C-Atomen, cyclischen oder verzweigten Alkyl-, Alkoxy- oder Thioalkylgruppen mit 3 bis 20 C-Atomen, Alkenyl- oder Alkinylgruppen mit 2 bis 20 C-Atomen, Hydroxyl-, Thiol-, Amino-, Hydrazin-, Nitro- oder Nitrilgruppen, unsubstituierten Aryl- oder Heteroarylgruppen wie bereits definiert, sowie Halogenen. Bevorzugte Substituenten der Arylreste in den Resten der Formeln -N(Ar)₂ und -N(HetAr)(Ar) sind Methyl-, Ethyl-, Propyl-, Isopropyl-, c-Propyl-, n-Butyl-, sec-Butyl-, Isobutyl- und t-Butylgruppen und ihre korrespondierenden Alkoxy- und Thioalkyl-Äquivalente. Die vorgenannten Arylreste können darüber hinaus mit einem oder mehreren Arylrest(en) der vorgenannten Arylreste oder mit einem oder mehreren Heteroarylrest(en) anneliert sein. Auch solche annelierten Aryl(Heteroaryl)-Systeme können wiederum substituiert oder unsubstituiert sein. Bevorzugte Substituenten sind dabei wie voranstehend definiert. Die vorgenannten Arylreste in den Formeln -N(Ar)₂ und -N(HetAr)(Ar) können auch über ihre jeweiligen Substituenten miteinander beziehungsweise mit dem Heteroaryl verbrückt sein. Bevorzugte Beispiele eines solchen verbrückten Diarylamins sind 9H-Acridin und 10,11-Dihydrobenzazepin. Auch diese verbrückten Diarylamine der Formel -N(Ar)₂ oder Aryl-Heteroarylamine der Formel -N(HetAr)(Ar) können substituiert oder unsubstituiert vorliegen, wobei bevorzugte Substituenten wie voranstehend definiert sind. Einige bevorzugte Beispiele eines substituierten verbrückten Diarylamins sind 9,9-Dimethylacridine, 9,9-Dihydroacridine, Carbazole, Iminodibenzyle, Phenoxazine, Phenothiazine, Iminostilbene (Dibenzazepine), Arylindoline, Arylbenzomorpholine, Arylbenzothiomorpholine oder Aryltetrahydrochinoline.

In verschiedenen Ausführungsformen der Verbindungen der Formel (la) handelt es sich bei den Heteroarylresten in den Resten der Formeln -N(HetAr)(Ar) und -N(HetAr)₂ unabhängig voneinander bevorzugt um substituiertes oder unsubstituiertes Furanyl, Difuranyl, Terfuranyl, Benzofuranyl, Isobenzofuranyl, Dibenzofuranyl, Thienyl, Dithienyl, Terthienyl, Benzothienyl, Isobenzothienyl, Benzodithienyl, Benzotrithienyl, Pyrrolyl, Indolyl, Isoindolyl, Carbazolyl, Pyridinyl, Chinolinyl, Isochinolinyl, Acridinyl, Phenanthridinyl, Benzo-5,6-chinolinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, Oxazolyl, Benzoxazolyl, Naphthoxazolyl, Anthroxazolyl, Phenanthroxazolyl, Isoxazolyl, 1,2-Thiazolyl, 1,3-Thiazolyl, Benzothiazolyl, Pyridazinyl, Benzopyridazinyl, Pyrimidinyl, Benzpyrimidinyl, Chinoxalinyl, Pyrazinyl, Phenazinyl, Naphthyridinyl, Carbazolyl, Benzocarbolinyl, Phenanthrolinyl, 1,2,3-Triazolyl, 1,2,4-Triazolyl, Benzotriazolyl, 1,2,3-Oxadiazolyl, 1,2,4-Oxadiazolyl, 1,2,5-Oxadiazolyl, 1,3,4-Oxadiazolyl, 1,2,3-Thiadiazolyl, 1,2,4-Thiadiazolyl, 1,2,5- Thiadiazolyl, 1,3,4-Thiadiazolyl, 1,3,5-Triazinyl, 1,2,4-Triazinyl, 1,2,3-Triazinyl, Tetrazolyl, 1,2,4,5-Tetrazinyl, 1,2,3,4-Tetrazinyl, 1,2,3,5-Tetrazinyl, Purinyl, Pteridinyl, Indolizinyl und Benzothiadiazolyl. Bei den Substituenten der vorgenannten Heteroarylreste handelt es sich beispielsweise um Substituenten ausgewählt aus der Gruppe bestehend aus Halogenen, substituierten oder unsubstituierten geradkettigen Alkyl-, Alkoxy- oder Thioalkylgruppen mit 1 bis 20 C-Atomen oder substituierten oder unsubstituierten, verzweigten oder cyclischen Alkyl-, Alkoxy- oder Thioalkylgruppen mit 3 bis 20 C-Atomen oder substituierten oder unsubstituierten Alkenyl- oder Alkinylgruppen mit 2 bis 20 C-Atomen handelt, wobei jede der vorgenannten Gruppen ein oder mehrere Heteroatome ausgewählt aus Si, O, S, Se, N und P beinhalten kann. Gesetz den Fall, dass die vorgenannten Substituenten der Heteroarylreste wiederum substituiert sind, sind deren Substituenten ausgewählt aus geradkettigen Alkyl-, Alkoxy- oder Thialkylgruppen mit 1 bis 20 C-Atomen, cyclischen oder verzweigten Alkyl-, Alkoxy- oder Thioalkylgruppen mit 3 bis 20 C-Atomen, Alkenyl- oder Alkinylgruppen mit 2 bis 20 C-Atomen, Hydroxyl-, Thiol-, Amino-, Hydrazin-, Nitro- oder Nitrilgruppen, unsubstituierten Aryl- oder Heteroarylgruppen wie bereits definiert, sowie Halogenen. Bevorzugte Substituenten der Heteroarylreste in den Resten der Formeln -N(HetAr)(Ar) und -N(HetAr)₂ sind Methyl-, Ethyl-, Propyl-, Isopropyl-, c-Propyl-, n-Butyl-, sec-Butyl-, Isobutyl- und t-Butylgruppen und ihre korrespondierenden Alkoxy- und Thioalkyl-Äquivalente. Die vorgenannten Heteroarylreste können darüber hinaus mit einem oder mehreren Heteroarylrest(en) der vorgenannten Heteroarylreste oder mit einem oder mehreren Arylrest(en) anneliert sein. Auch solche annelierten Aryl(Heteroaryl)-Systeme können wiederum substituiert oder unsubstituiert sein. Die vorgenannten Heteroarylreste in den Formeln - N(HetAr)(Ar) und -N(HetAr)₂ können auch über ihre jeweiligen Substituenten miteinander beziehungsweise mit dem Aryl verbrückt sein. Auch diese verbrückten Diarylamine der Formel -N(Ar)₂ oder Aryl-Heteroarylamine der Formel -N(HetAr)(Ar) können substituiert oder unsubstituiert vorliegen, wobei bevorzugte Substituenten wie voranstehend definiert sind.

In einem Aspekt betrifft die Erfindung eine Verbindung der allgemeinen Formel (II):

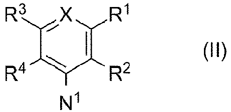

In der Verbindung gemäß Formel (II) bezeichnet X C-CF₃, C-CCl₃, C-Cl, C-SCN oder C-CN; N¹ bezeichnet einen Stickstoff-haltigen Heteroarylrest, der über ein Stickstoffatom gebunden ist, oder einen Rest der Formel -N(Ar)₂, -N(HetAr)(Ar), -N(HetAr)₂, wobei jedes Ar unabhängig ein Arylrest und jedes HetAr unabhängig ein Heteroarylrest ist; und R¹, R², R³ und R⁴ bezeichnen jeweils unabhängig voneinander F, Cl, Br, CF₃, SCN, CN oder H, mit der Maßgabe, dass nur ein Substituent R¹⁻⁴ CN ist, und dass, wenn X C-CN ist, keiner der Substituenten R¹⁻⁴ CN ist.

In verschiedenen Ausführungsformen der Verbindungen der Formel (II) gelten für den Stickstoff-haltigen Heteroarylrest (N¹) die gleichen bevorzugten Auswahlkriterien wie voranstehend für Verbindungen der Formel (Ia) definiert.

In verschiedenen Ausführungsformen der Verbindungen der Formel (II) gelten darüber hinaus für Arylreste in den Resten der Formeln -N(Ar)₂ und -N(HetAr)(Ar) und für Heteroarylreste in den Resten der Formeln -N(HetAr)(Ar) und -N(HetAr)₂ die gleichen Auswahlkriterien wie voranstehend für Verbindungen der Formel (Ia) definiert.

In verschiedenen Ausführungsformen gelten für die Substituenten der Verbindung der Formel (II) folgende Definitionen:
Der Substituent X bezeichnet bevorzugt C-CF₃ oder C-CN; der Substituent N¹ bezeichnet bevorzugt einen substituierten oder unsubstituierten Phenoxazin-, Phenothiazin-, Indol-, Benzimidazol-, Chinolin-, Dibenzazepin-, Carbazol- oder 9H-Acridinrest, der jeweils über das Stickstoffatom an den Aromaten der Formel (II) gebunden ist, ganz besonders bevorzugt einen Phenoxazin, Phenothiazin, 2-Phenylindolin-, 2-Phenylbenzimidazol-, 2-Naphthylbenzimidazol-, 2-Phenyl-1,2,3,4-tetrahydrochinolin-, Dibenzazepin-, 10,11-Dihydrobenzazepin-, 2,3-Indolocarbazol-, 2,3-(1-Methylindolo)carbazol-, 9-H-Acridin- oder 9,9-Dimethylacridinrest, der jeweils über das Stickstoffatom des Hauptkohlenstoffrings an den Aromaten der Formel (II) gebunden ist; der Substituent R² bezeichnet bevorzugt CF₃ oder F; und die Substituenten R¹, R³ und R⁴ bezeichnen bevorzugt F oder H.

Konkrete Ausführungsformen der Verbindungen der allgemeinen Formel (II) sind die Verbindungen 1a-1y und 4F1Cz:

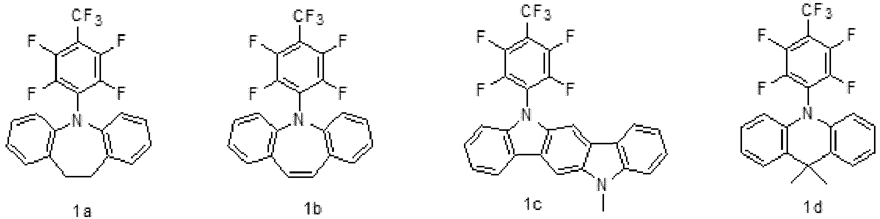

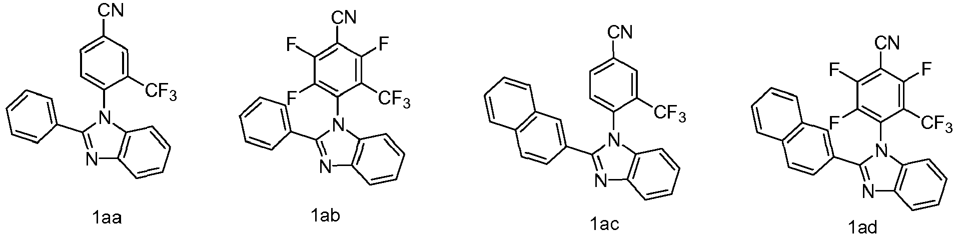

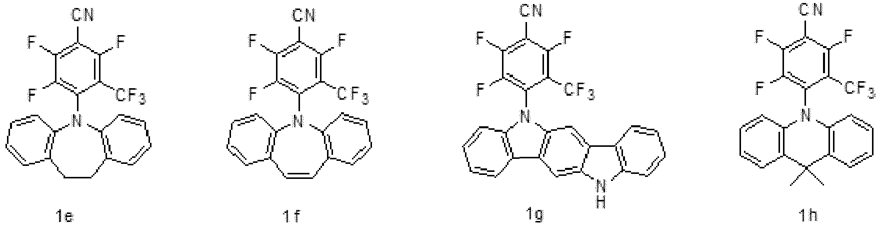

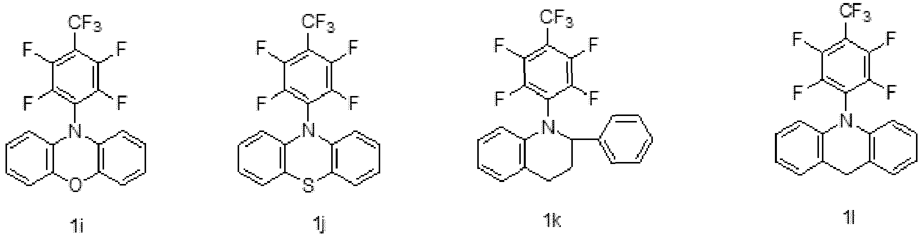

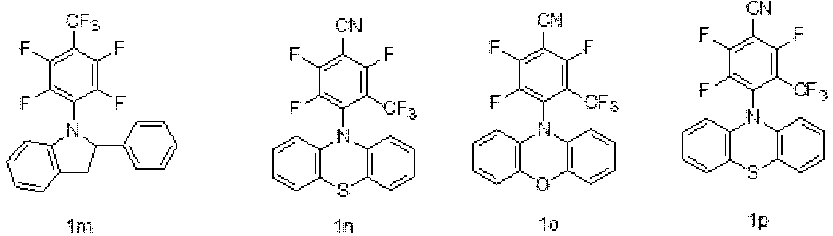

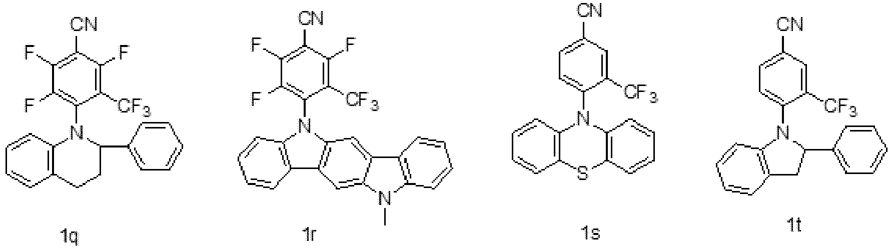

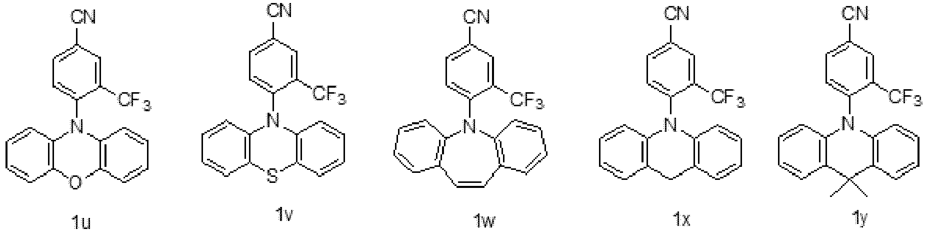

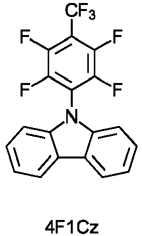

In einem weiteren Aspekt betrifft die Erfindung Verbindungen der allgemeinen Formel (III):

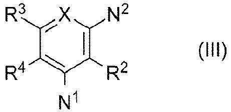

In der Verbindung der allgemeinen Formel (III) bezeichnet X C-CF₃, C-SCN, C-CCl₃, C-Cl, C-F, C-CN oder N; N¹ und N² bezeichnen jeweils unabhängig voneinander einen Stickstoff-haltigen Heteroarylrest, der über ein Stickstoffatom gebunden ist, oder einen Rest der Formel -N(Ar)₂, - N(HetAr)(Ar), -N(HetAr)₂, wobei jedes Ar unabhängig ein Arylrest und jedes HetAr unabhängig ein Heteroarylrest ist; und R², R³ und R⁴ bezeichnen jeweils unabhängig voneinander F, Cl, Br, C1-C12 Perfluoralkyl, CF₃, SCN, CN oder H, mit der Maßgabe, dass nur ein Substituent R^{2,3,4} CN ist, und dass, wenn X C-CN ist, keiner der Substituenten R^{2,3,4} CN ist.

In verschiedenen Ausführungsformen der Verbindungen der Formel (III) gelten für den Stickstoff-haltigen Heteroarylrest (N^{1,2}) die gleichen bevorzugten Auswahlkriterien wie voranstehend für Verbindungen der Formel (Ia) definiert.

In verschiedenen Ausführungsformen der Verbindungen der Formel (III) gelten darüber hinaus für Arylreste in den Resten der Formeln -N(Ar)₂ und -N(HetAr)(Ar) und für Heteroarylreste in den Resten der Formeln -N(HetAr)(Ar) und -N(HetAr)₂ die gleichen Auswahlkriterien wie voranstehend für Verbindungen der Formel (Ia) definiert.

In verschiedenen Ausführungsformen gelten für die Substituenten der Verbindung der Formel (III) folgende Definitionen:
Der Substituent X bezeichnet bevorzugt C-CF₃, C-Cl, oder N; die Substituenten N¹ und N² bezeichnen bevorzugt jeweils unabhängig voneinander einen substituierten oder unsubstituierten Chinolin-, Benzimidazol-, Phenoxazin-, Benzomorpholin-, Benzothiomorpholin-, Dibenzazepin-, Carbazol- oder 9H-Acridinrest, der jeweils über das Stickstoffatom an den Aromaten der Formel (III) gebunden ist, ganz besonders bevorzugt einen 2-Phenyl-1,2,3,4-tetrahydrochinolin-, 2-Phenylbenzimidazol-, Phenoxazin-, 3-Phenylbenzomorpholin-, 3-Phenylbenzothiomorphlolin-, Dibenzazepin-, 10,11-Dihydrobenzazepin-, 2,3-Indolocarbazol-, 2,3-(1-Methylindolo)carbazol-, 9H-Acridin- oder 9,9-Dimethylacridinrest, der jeweils über das Stickstoffatom des Hauptkohlenstoffrings an den Aromaten der Formel (III) gebunden ist; der Substituent R² bezeichnet bevorzugt CN, CF₃, F oder Cl; und die Substituenten R³ und R⁴ bezeichnen bevorzugt H, CN, CF₃, F oder Cl.

Konkrete Ausführungsformen der Verbindungen der allgemeinen Formel (III) sind die Verbindungen 2a-2t und 3F2Cz:

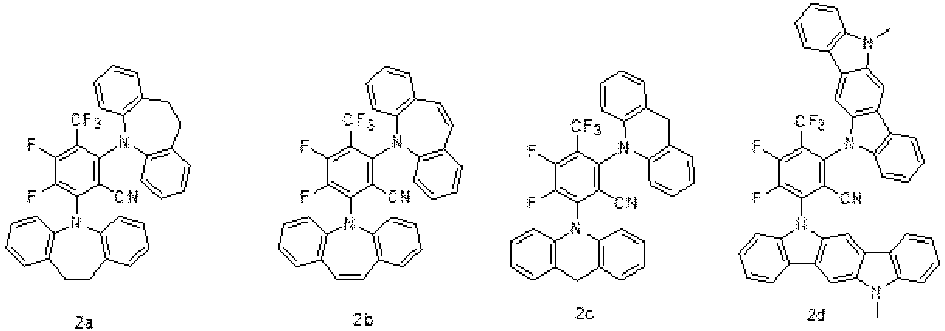

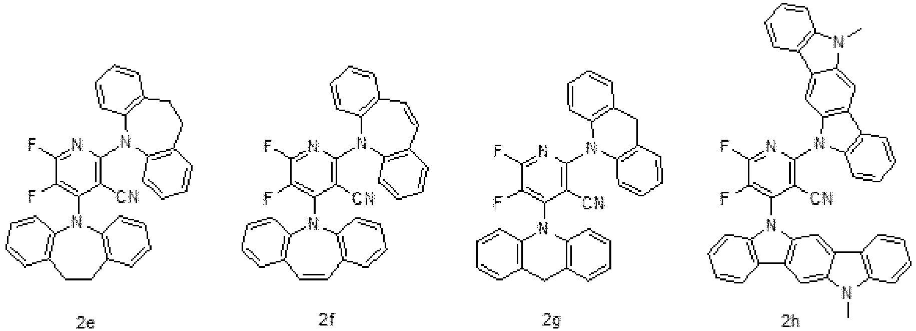

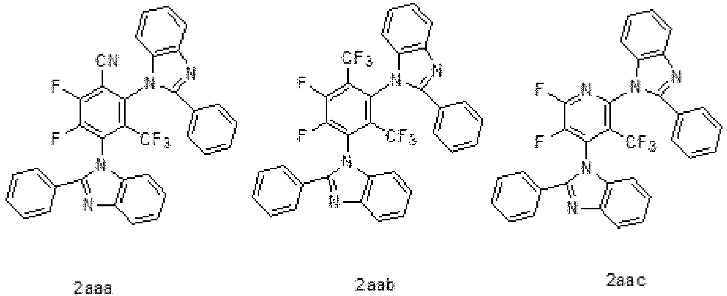

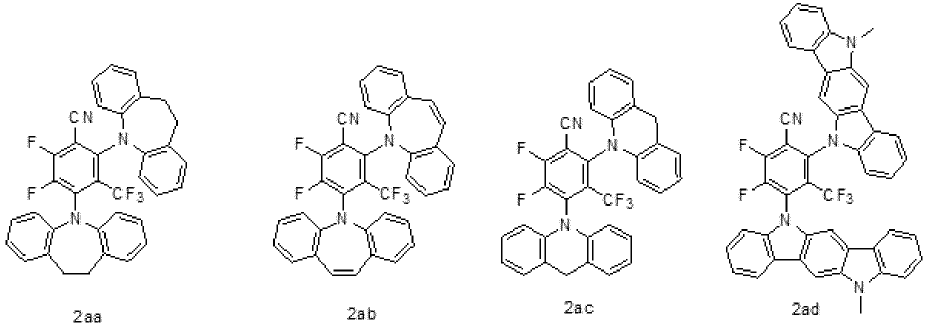

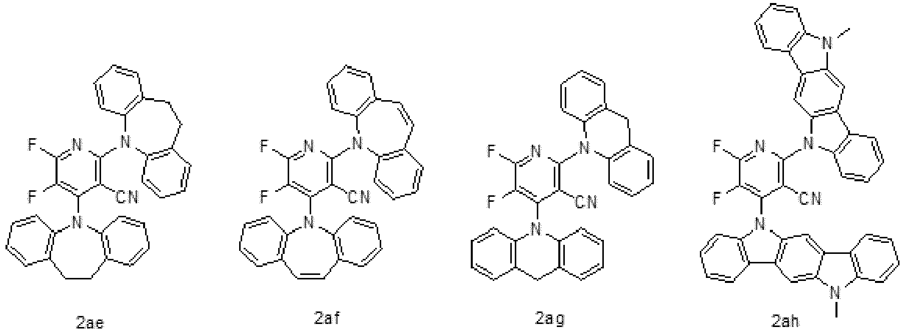

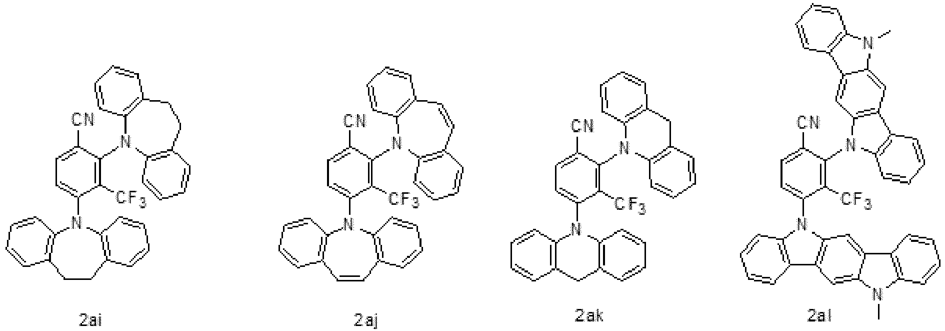

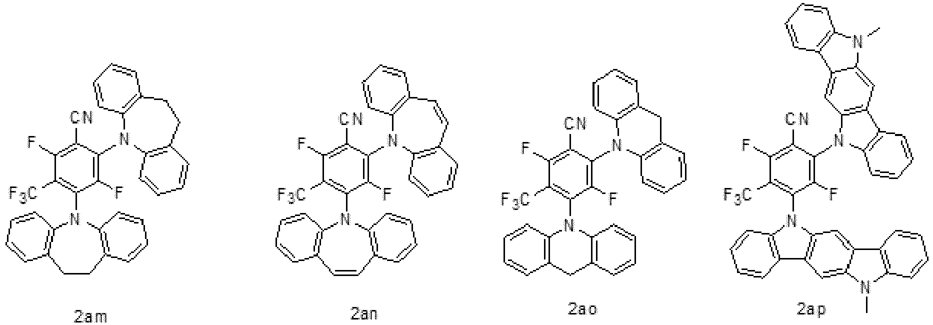

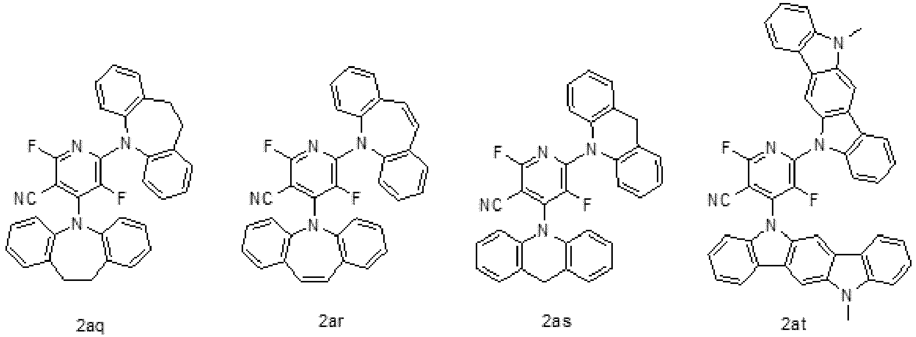

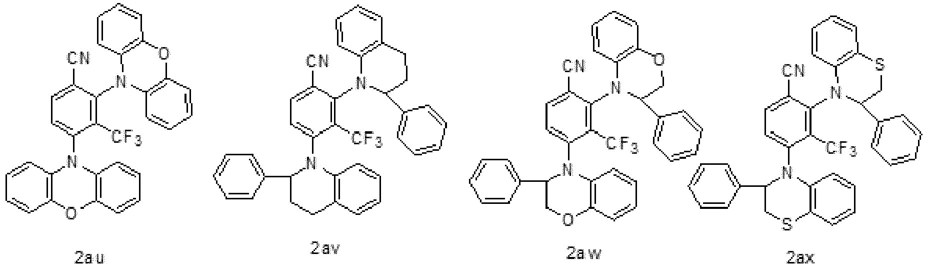

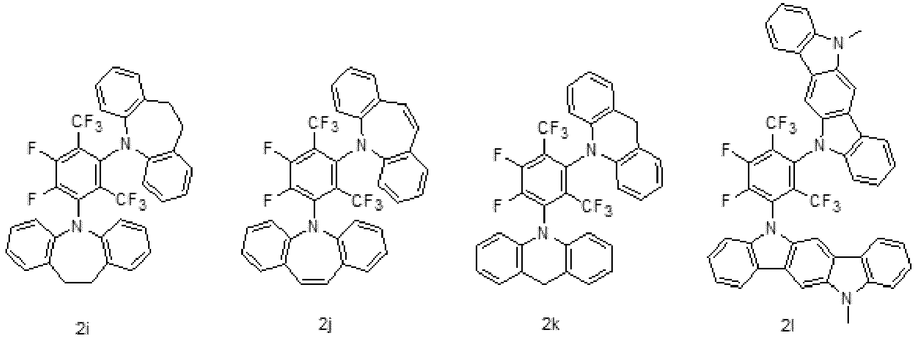

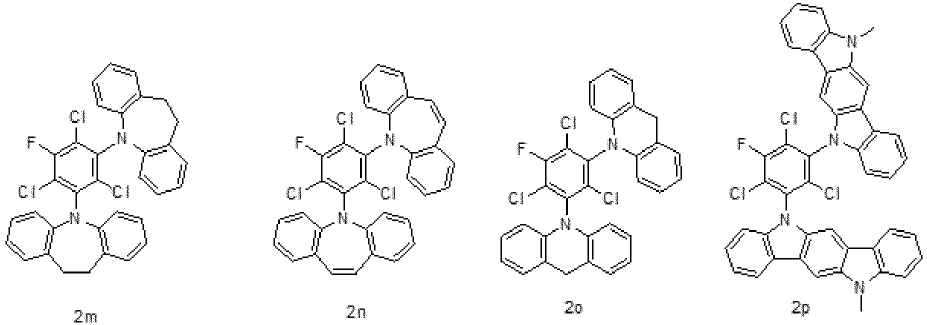

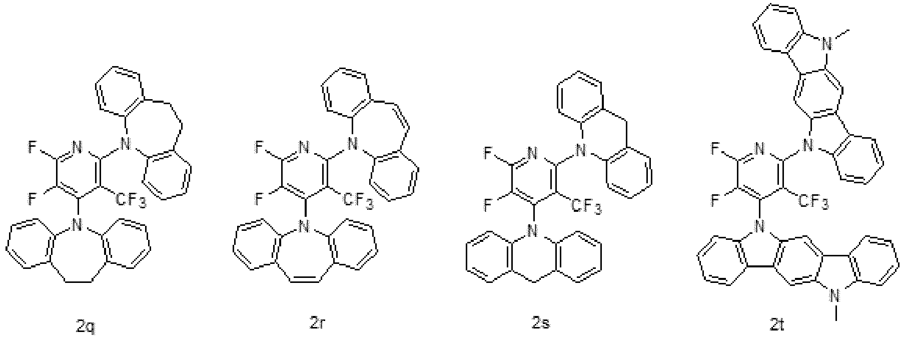

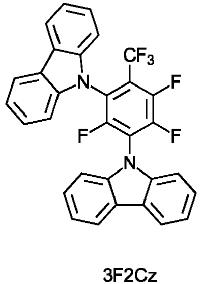

In einem weiteren Aspekt betrifft die vorliegende Erfindung Verbindungen der allgemeinen Formel (IV):

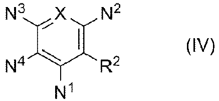

In der Verbindung der allgemeinen Formel (IV) bezeichnet X C-CF₃, C-Cl₃, C-Cl, C-F, C-SCN, C-CN oder N; bezeichnet R² F, Cl, Br, CF₃, SCN, CN oder H, mit der Maßgabe, dass R² nicht CN ist, wenn X C-CN darstellt; und bezeichnen N¹, N², N³ und N⁴ jeweils unabhängig voneinander einen Stickstoff-haltigen Heteroarylrest, der über ein Stickstoffatom gebunden ist, oder einen Rest der Formel -N(Ar)₂, -N(HetAr)(Ar), -N(HetAr)₂, wobei jedes Ar unabhängig ein Arylrest und jedes HetAr unabhängig ein Heteroarylrest ist.

In verschiedenen Ausführungsformen der Verbindungen der Formel (IV) gelten für den Stickstoff-haltigen Heteroarylrest (N¹⁻⁴) die gleichen bevorzugten Auswahlkriterien wie voranstehend für Verbindungen der Formel (la) definiert.

In verschiedenen Ausführungsformen der Verbindungen der Formel (IV) gelten darüber hinaus für Arylreste in den Resten der Formeln -N(Ar)₂ und -N(HetAr)(Ar) und für Heteroarylreste in den Resten der Formeln -N(HetAr)(Ar) und -N(HetAr)₂ die gleichen Auswahlkriterien wie voranstehend für Verbindungen der Formel (Ia) definiert.

In verschiedenen Ausführungsformen gelten für die Substituenten der Verbindung der Formel (IV) folgende Definitionen:
Der Substituent X bezeichnet bevorzugt C-CN, C-CF₃, C-Cl oder N; die Substituenten N¹⁻⁴ bezeichnen bevorzugt jeweils unabhängig voneinander einen substituierten oder unsubstituierten Chinolin-, Benzomorpholin-, Benzothiomorpholin-, Indol-, Benzimidazol-, Phenoxazin-, Phenothiazin-, Dibenzazepin-, Carbazol- oder 9H-Acridinrest, der jeweils über das Stickstoffatom an den Aromaten der Formel (IV) gebunden ist, ganz besonders bevorzugt einen 2-Phenylindol-, 2-Phenylbenzimidazol-, 1,4-Dihydro-2-phenylchinolin-, 3-Phenyl-1,4-benzoxazin-, 3-Phenyl-1,4-benzothiazin-, Phenoxazin-, Phenothiazin-, Dibenzazepin-, 10,11-Dihydrobenzazepin-, 2,3-Indolocarbazol-, 2,3-(1-Methylindolo)carbazol-, oder 9,9-Dimethylacridinrest, der jeweils über das Stickstoffatom des Hauptkohlenstoffrings an den Aromaten der Formel (IV) gebunden ist; und der Substituent R² bezeichnet bevorzugt CF₃, SCN, F oder CN.

Konkrete Ausführungsformen der Verbindungen der allgemeinen Formel (IV) sind die Verbindungen 3a-3lf, 4aaa-4aae und 1F4Cz:

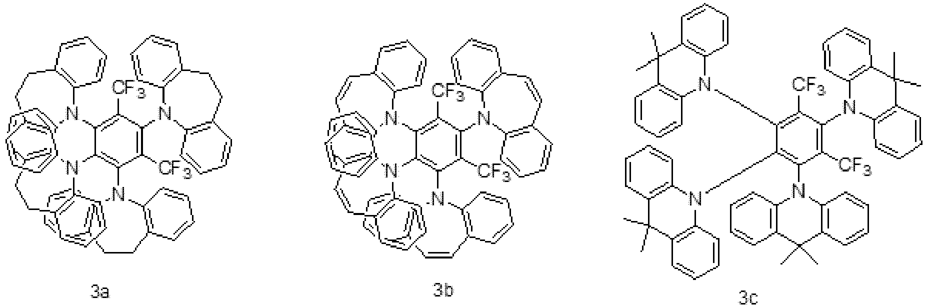

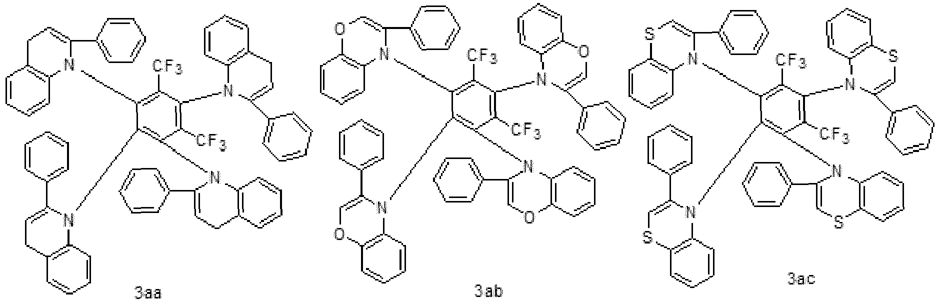

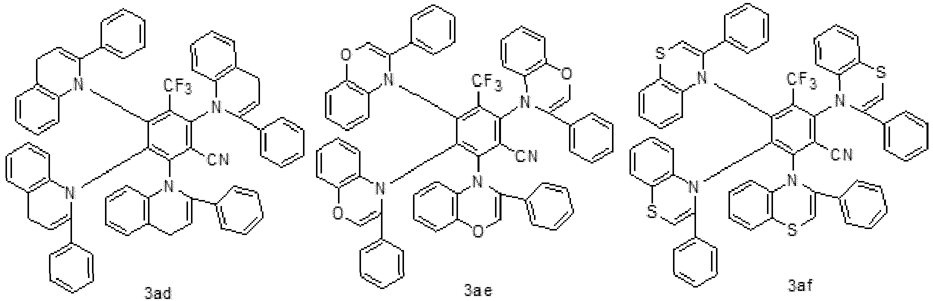

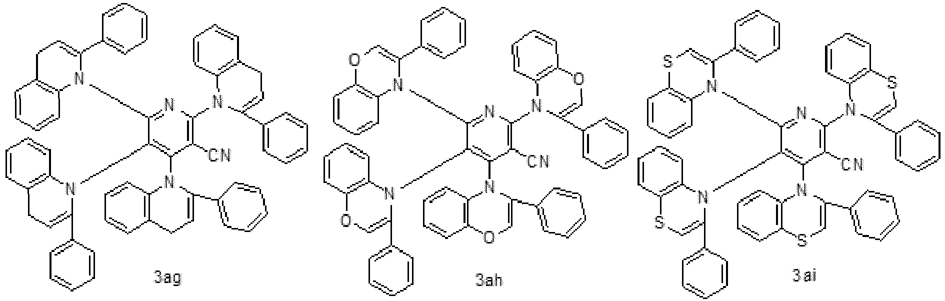

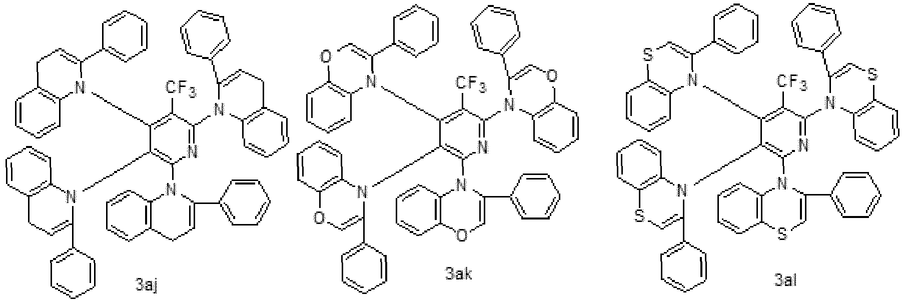

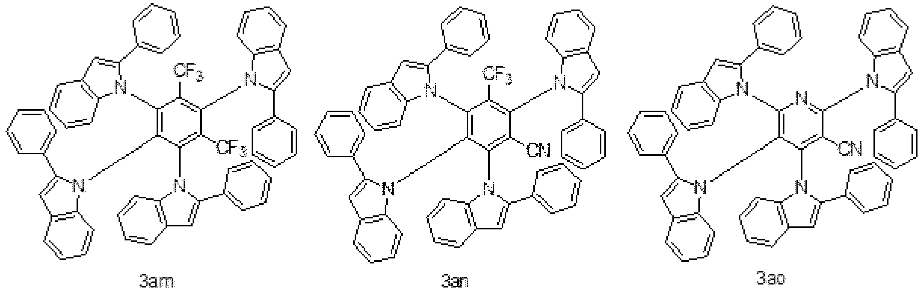

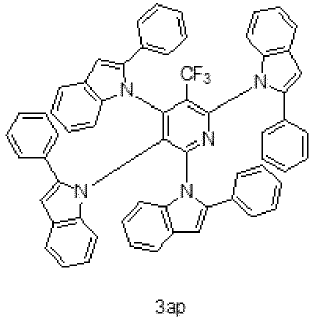

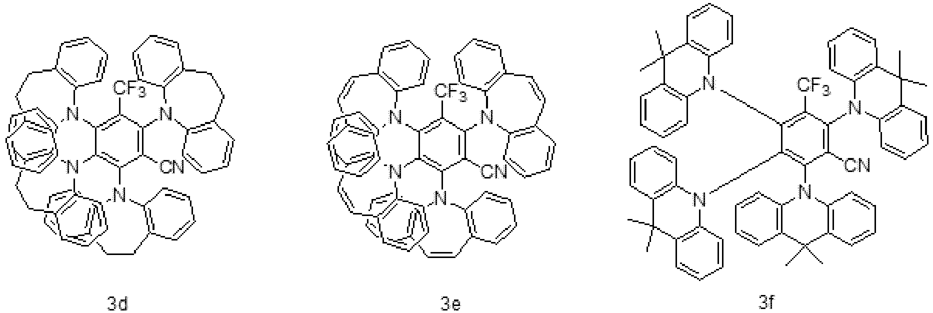

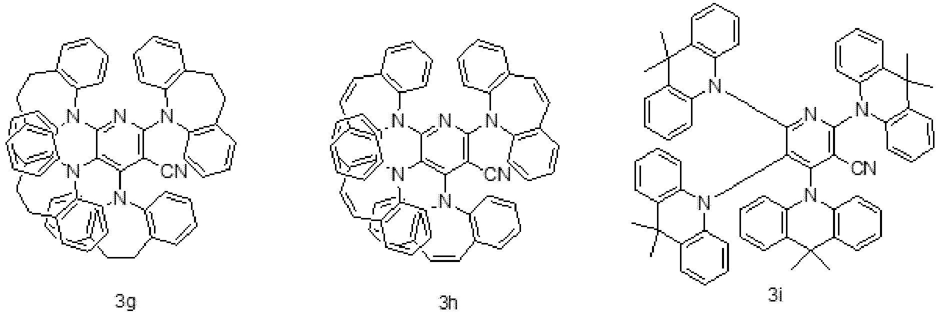

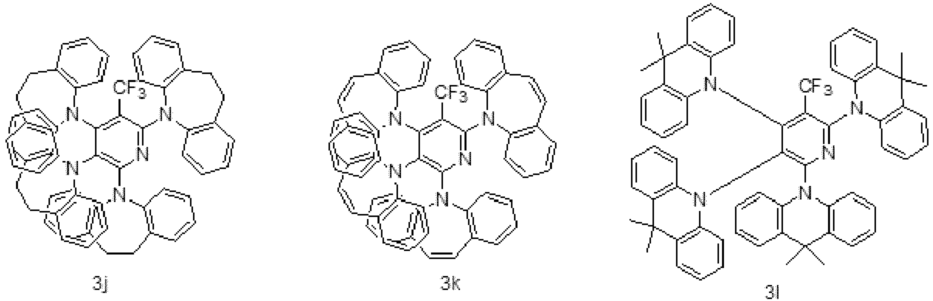

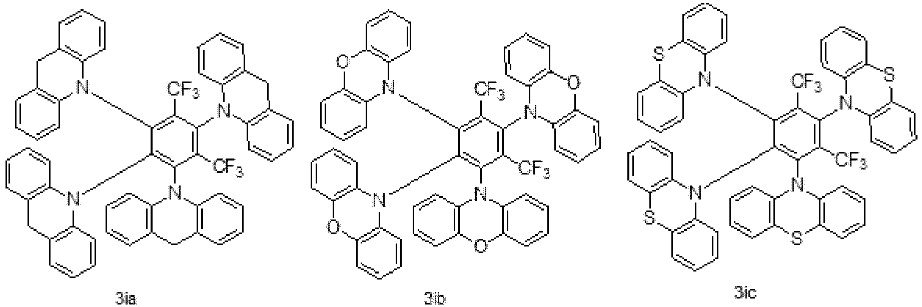

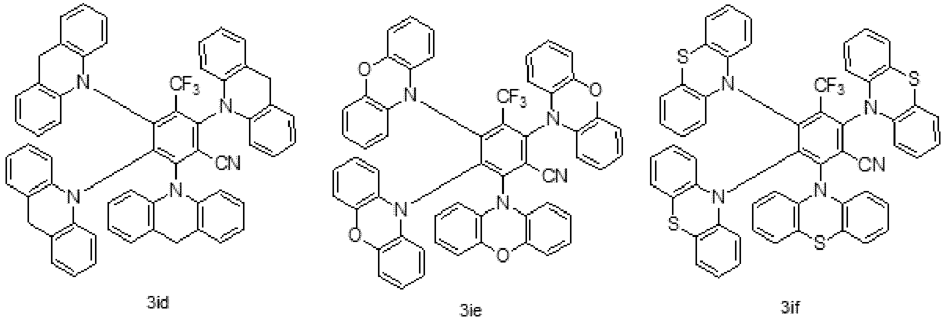

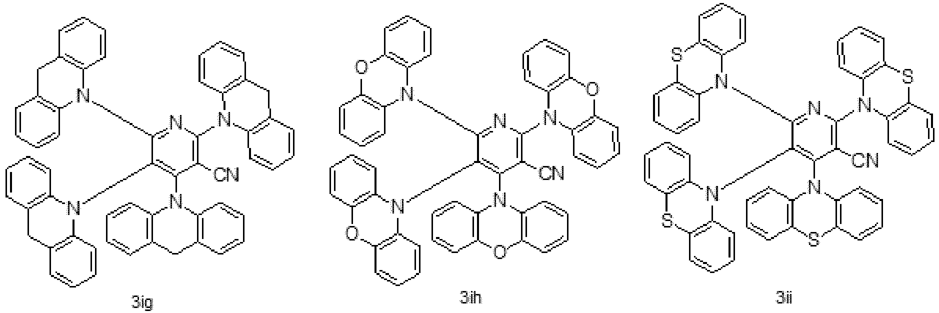

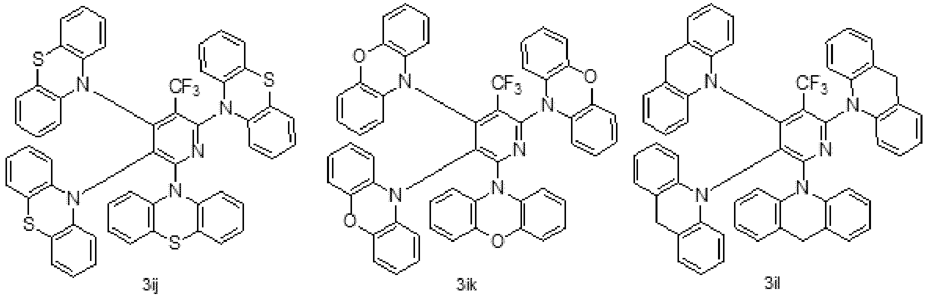

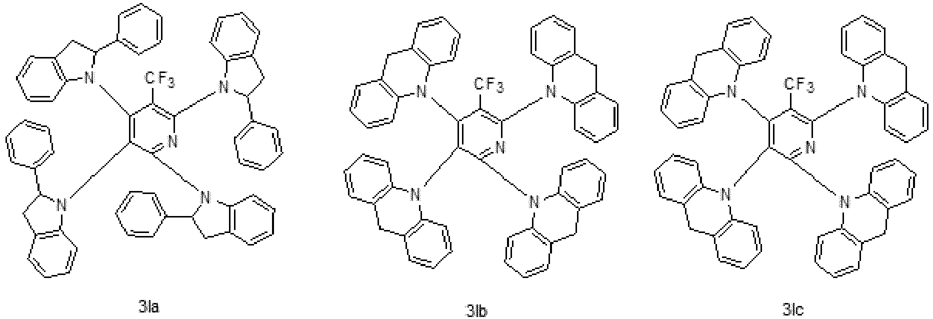

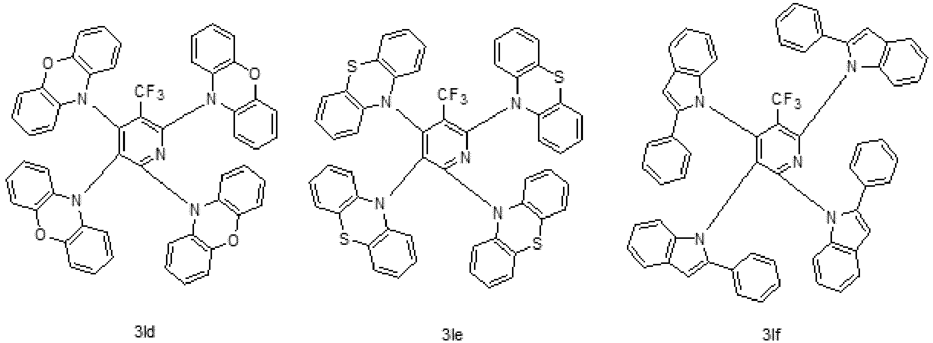

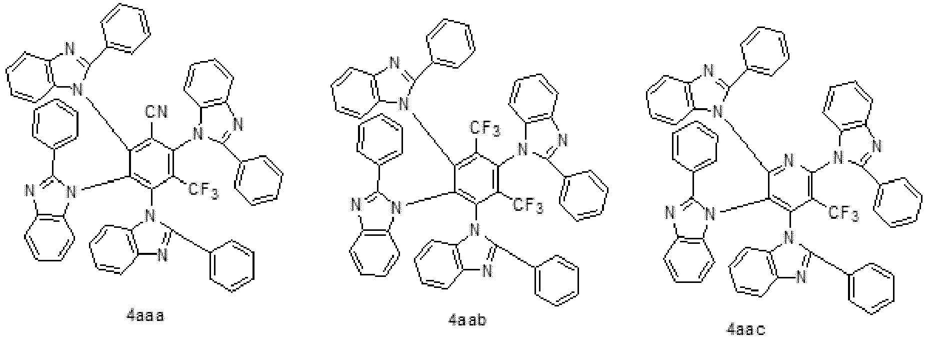

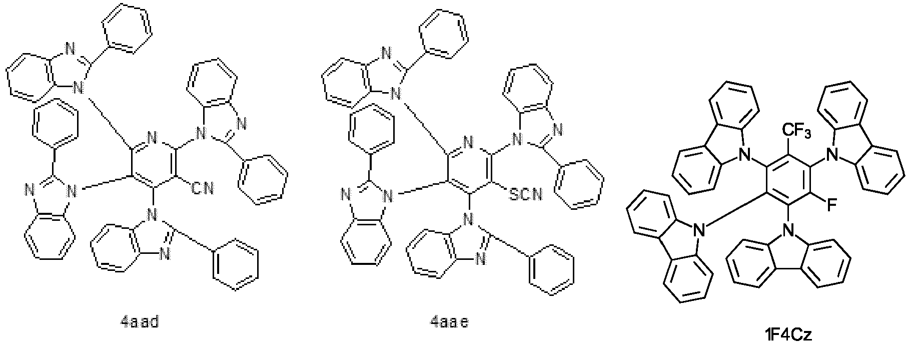

In verschiedenen Ausführungsformen sind die Verbindungen der Formel (IV) auf solche Verbindungen beschränkt, in denen, wenn X C-CN ist und N¹, N², N³ und N⁴ jeweils ein über das Stickstoffatom gebundenes Carbazol sind, R² nicht F ist.

In verschiedenen Ausführungsformen handelt es sich bei dem bei dem optoelektronischen Bauelement um OSCs mit einer photoaktiven organischen Schicht. Diese photoaktive Schicht beinhaltet niedermolekulare Verbindungen, Oligomere, Polymere oder Mischungen hiervon als organische Beschichtungsmaterialien. Auf diesem Dünnschichtbauelement ist eine vorzugsweise opake oder semitransparente Elektrode als Deckkontaktlage aufgebracht.

Nach einer weiteren Ausführungsform der Erfindung ist das optoelektronische Bauelement auf einem flexibel ausgeführten Substrat angeordnet.

Unter einem flexiblen Substrat wird im Sinne der vorliegenden Erfindung ein Substrat verstanden, welches eine Verformbarkeit infolge äußerer Krafteinwirkung gewährleistet. Dadurch sind solche flexiblen Substrate auch zur Anordnung auf gekrümmten Oberflächen geeignet. Flexible Substrate schließen beispielsweise Plastik- oder Metallfolien ein, ohne darauf beschränkt zu sein.

In verschiedenen Ausführungsformen erfolgt die Beschichtung zur Herstellung eines optoelektronischen Bauelements mittels Vakuumprozessierung der erfindungsgemäßen organischen Verbindungen. In verschiedenen Ausführungsformen weisen die verwendeten erfindungsgemäßen Verbindungen zur Herstellung des optoelektronischen Bauelements daher eine niedrige Verdampfungstemperatur, bevorzugt <300°C, besonders bevorzugt <250°C, aber nicht niedriger als 150 °C, auf. In verschiedenen Ausführungsformen beträgt die Verdampfungstemperatur aber mindestens 120°C. Besonders vorteilhaft ist es, wenn die erfindungsgemäßen organischen Verbindungen im Hochvakuum sublimierbar sind.

Nach einer weiteren Ausgestaltung der vorliegenden Erfindung kann vorgesehen sein, dass die Beschichtung zur Herstellung eines optoelektronischen Bauelements mittels Lösungsmittelprozessierung der hierin beschriebenen Verbindungen erfolgt. Durch die Verfügbarkeit kommerzieller Sprühroboter ist dieses Auftragsverfahren vorteilhaft einfach auf industrielle Maßstäbe Rolle-zu-Rolle skalierbar.

In verschiedenen Ausführungsformen handelt es sich bei dem optoelektronischen Bauelement im Sinne der vorliegenden Erfindung um eine gattungsgemäße Solarzelle. Ein solches optoelektronisches Bauelement besitzt üblicherweise einen Schichtaufbau, wobei die jeweils unterste und oberste Lage als Elektrode und Gegenelektrode zur elektrischen Kontaktierung ausgebildet sind. In verschiedenen Ausführungsformen wird das optoelektronische Bauelement auf einem Substrat, wie beispielsweise Glas, Kunststoff (PET, etc.) oder einem Metallband angeordnet. Zwischen der substratnahen Elektrode und der Gegenelektrode ist zumindest eine organische Schicht umfassend zumindest eine organische Verbindung angeordnet. Als organische Verbindung können hierbei organische niedermolekulare Verbindungen, Oligomere, Polymere oder Mischungen verwendet werden. In verschiedenen Ausführungsformen handelt es sich bei der organischen Schicht um eine photoaktive Schicht. In verschiedenen Ausführungsformen der photoaktiven Schicht kann beispielsweise in Form einer Mischschicht aus einem Elektronen-Donor- und einem Elektronen-Akzeptormaterial ausgebildet. Angrenzend zu der zumindest einen photoaktiven Schicht können Ladungsträgertransportschichten angeordnet sein. Diese können je nach Ausgestaltung vorzugsweise Elektronen (= negative Ladungen) oder Löcher (= positive Ladungen) von oder zu den jeweiligen Elektroden transportieren. In verschiedenen Ausführungsformen ist das optoelektronische Bauelement als Tandem- oder Mehrfachbauelement ausgestaltet. Dabei werden zumindest zwei optoelektronische Bauelemente als Schichtsystem übereinander abgeschieden. Auf bzw. unter den als Kontakt ausgebildeten Grund- und Decklagen können sich in verschiedenen Ausführungsformen zusätzliche Schichten zur Beschichtung oder Verkapselung des Bauelements oder weitere Bauelemente anschließen.

In einer Ausführungsform der Erfindung ist die organische Schicht als eine oder mehrere dünne Lagen vakuumprozessierter niedermolekularer Verbindungen oder organischer Polymere ausgebildet. Aus dem Stand der Technik ist eine Vielzahl auf vakuumprozessierten niedermolekularer Verbindungen und Polymeren basierender, optoelektronischer Bauelemente bekannt (Walzer et al., Chemical reviews 2007, 107(4), 1233-1271; Peumans et al., J. Appl. Phys. 2003, 93(7), 3693-3722).

Bevorzugt wird die organische Schicht unter Verwendung vakuumprozessierbarer Verbindungen der hierin beschriebenen erfindungsgemäßen Verbindungen auf einem Substrat abgeschieden. Nach einer alternativ bevorzugten Ausgestaltung der vorliegenden Erfindung wird die organische Schicht unter Verwendung von Lösungen nasschemisch auf einem Substrat abgeschieden.

Typische Beispiele für optoelektronische Bauelemente, die erfindungsgemäße Verbindungen wie oben beschrieben enthalten, werden ebenfalls bereitgestellt. In derartigen Ausführungsformen wird die erfindungsgemäße Verbindung in verschiedenen Ausführungsformen ausgewählt aus der Gruppe bestehend aus Verbindungen 1a-1y, 2a-2t, 3a-3lf, 4F1Cz, 3F2Cz, 2F3Cz und 1F4Cz wie oben definiert.

In verschiedenen anderen Ausführungsformen der vorliegenden Erfindung handelt es sich bei dem optoelektronischen Bauelement, welches mindestens eine der Verbindungen der Formel (I) wie hierin beschrieben enthält, um eine organische Leuchtdiode (OLED).

Die erfindungsgemäßen OLEDs sind grundsätzlich aus mehreren Schichten aufgebaut, z.B.:
1. Anode
2. Lochleiterschicht
3. Licht-emittierende Schicht
4. Blockschicht für Löcher/Excitonen
5. Elektronenleiterschicht
6. Kathode

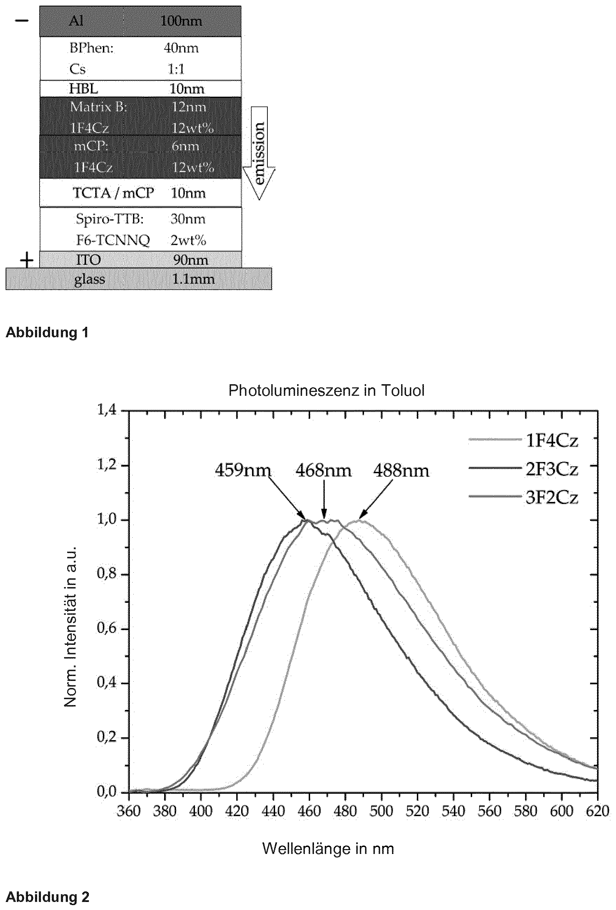

Es sind auch von dem vorstehend genannten Aufbau verschiedene Schichtenfolgen möglich, die dem Fachmann bekannt sind. Beispielsweise ist es möglich, dass das OLED nicht alle der genannten Schichten aufweist, zum Beispiel ist eine OLED mit den Schichten (1) (Anode), (3) (Licht-emittierende Schicht) und (6) (Kathode) ebenfalls geeignet, wobei die Funktionen der Schichten (2) (Lochleiterschicht) und (4) (Blockschicht für Löcher/Excitonen) und (5) (Elektronenleiterschicht) durch die angrenzenden Schichten übernommen werden. OLEDs, die die Schichten (1), (2), (3) und (6) bzw. die Schichten (1), (3), (4), (5) und (6) aufweisen, sind ebenfalls geeignet. Des Weiteren können die OLEDs zwischen der Anode (1) und der Lochleiterschicht (2) eine Blockschicht für Elektronen/Excitonen aufweisen. Der Aufbau einer OLED ist in Abb. 1 schematisch gezeigt (BPhen Cs = Bathophenanthrolin mit Cs dotiert; HBL = Löcher-blockierende Schicht, mCP = 1,3-Bis(N-carbazolyl)benzen, Spiro-TTB = 2,2',7,7'-tetra(N,N-ditolyl)amino-9,9-spiro-bifluoren, TCTA = Tris(4-Carbazoyl-9-ylphenyl)amin, F6-TCNNQ = 2,2-(perfluoronaphthalen-2,6-diyliden)dimalononitril, ITO =) Indiumzinnoxid, Al = Aluminium, 1F4Cz = 9,9',9",9"'-(2-Fluoro-6-(trifluoromethyl)benzo-1,3,4,5-tetrayl)tetrakis(9H-carbazol)).

Die Verbindungen der Formeln (II), (III) und (IV) können als Emitter- oder Matrixmaterialien in der Licht-emittierenden Schicht Verwendung.

Die Verbindungen der Formeln (II), (III) und (IV) können als alleiniges Emitter-und/oder Matrixmaterial - ohne weitere Zusätze - in der Licht-emittierenden Schicht vorliegen. Es ist jedoch ebenfalls möglich, dass neben den erfindungsgemäß eingesetzten Verbindungen der Formeln (II), (III) und (IV) weitere Verbindungen in der Licht-emittierenden Schicht vorliegen. Beispielsweise können ein oder mehrere fluoreszierende Farbstoffe anwesend sein, um die Emissionsfarbe des vorhandenen Emittermoleküls zu verändern. Des Weiteren kann ein Verdünnungsmaterial eingesetzt werden. Dieses Verdünnungsmaterial kann ein Polymer sein, zum Beispiel Poly(N-vinylcarbazol) oder Polysilan. Das Verdünnungsmaterial kann jedoch ebenfalls ein kleines Molekül sein, zum Beispiel 4,4'-N,N'-Dicarbazolbiphenyl (CBP = CDP) oder tertiäre aromatische Amine.

Die einzelnen der vorstehend genannten Schichten des OLEDs können wiederum aus 2 oder mehreren Schichten aufgebaut sein. Beispielsweise kann die Löcher-transportierende Schicht aus einer Schicht aufgebaut sein, in die aus der Elektrode Löcher injiziert werden, und einer Schicht, die die Löcher von der Loch injizierenden Schicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten organischen Verbindungen angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der Elektronentransportierenden Schicht sollte mit der Arbeitsfunktion der Kathode angeglichen sein.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen 11, 4 und 5 des Periodensystems der Elemente sowie die Übergangsmetalle der Gruppen 9 und 10. Wenn die Anode lichtdurchlässig sein soll, werden im Allgemeinen gemischte Metalloxide der Gruppen 12, 13 und 14 des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können. Bevorzugt wird als Material für die Anode (1) ITO eingesetzt.

Geeignete Lochleitermaterialien für die Schicht (2) der erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus tris-[N-(1-Naphthyl)-N-(phenylamino)]triphenylamin (1-NaphDATA), 4,4'-Bis[N-(1-naphthyl)-N-phenyl-amino]biphenyl (α-NPD), N,N'-Diphenyl-N,N'-bis(3-methylphenyl)-[1,1'-biphenyl]-4,4'-diamin (TPD),1,1-Bis[(di-4-tolylamino)phenyl]-cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-Tetrakis(4-methylphenyl)-(1,1'-biphenyl)-4,4'-diamin (TTB), 4,4',4"-tris(N,N-Diphenylamino)triphenylamin (TDTA), Porphyrinverbindungen und Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können in verschiedenen Ausführungsformen Carben-Komplexe als Lochleitermaterialien eingesetzt werden, wobei die Bandlücke des mindestens einen Lochleitermaterials im Allgemeinen größer ist als die Bandlücke des eingesetzten Emittermaterials. Dabei ist unter Bandlücke im Sinne der vorliegenden Anmeldung die Triplett-Energie zu verstehen. Geeignete Carben-Komplexe sind z.B. Carben-Komplexe, wie sie in WO 2005/019373 A2, WO 2006/056418 A2 und WO 2005/113704 und in den älteren, nicht vorveröffentlichten Europäischen Anmeldungen EP 06112228.9 und EP 06112198.4 beschrieben sind.

Die Licht-emittierende Schicht (3) enthält mindestens ein Emittermaterial. Dabei kann es sich grundsätzlich um einen Fluoreszenz oder Phosphoreszenzemitter handeln, wobei geeignete Emittermaterialien dem Fachmann bekannt sind. Bevorzugt handelt es sich bei dem mindestens einen Emittermaterial um einen Phosphoreszenzemitter. Dabei handelt es sich bei mindestens einem der in der Licht-emittierenden Schicht (3) enthaltenden Emittermaterialien um eine Verbindung der Formeln (II), (III) und (IV) wie hierin beschrieben. Darüber hinaus kann mindestens eine Verbindung der Formeln (II), (III) und (IV) zusätzlich als Matrixmaterial eingesetzt werden. Alternativ sind im Stand der Technik üblicherweise gebräuchliche Matrixmaterialien dem Fachmann bekannt. Beispielhafte Matrixmaterialien sind ausgewählt aus den Klassen der Oligoarylene (z.B. 2,2',7,7'-Tetraphenylspirobifluoren oder Dinaphthylanthracen), insbesondere der Oligoarylene enthaltend annelierte aromatische Gruppen wie z.B. Anthracen, Benzanthracen, Benzphenanthren, Phenanthren, Tetracen, Coronen, Chrysen, Fluoren, Spirofluoren, Perylen, Phthaloperylen, Naphthaloperylen, Decacyclen, Rubren, der Oligoarylenvinylene (z.B. DPVBi = 4,4'-Bis(2,2-diphenylethenyl)-1,r-biphenyl) oder Spiro-DPVBi gemäß EP 676461), oder der polypodalen Metallkomplexe, insbesondere Metallkomplexe von 8-Hydroxychinolin, z.B. AlQ₃ (= Aluminium(III)tris(8-hydroxychinolin)) oder Bis(2-methyl-8-chinolinolato)-4-(phenylphenolinolato)-aluminium. Generell sind geeignete Matrix-Materialien dem Fachmann z.B. durch Organic Light-Emitting Materials and Devices (Optical Science and Engineering Series; Ed.: Z. Li, H. Meng, CRC Press Inc., 2006 publiziert) bekannt.

Die Blockschicht für Löcher/Excitonen (4) kann üblicherweise in OLEDs eingesetzte Lochblockermaterialien aufweisen wie 2,9-Dimethyl-4,7-diphenyl-1,10-phenanthrolin (Bathocuproin, (BCP)), Bis-(2-methyl-8-chinolinato)-4-phenyl-phenylato)-aluminium(III) (BAlq), Phenothiazin-S,S-dioxidderivate und 1,3,5-tris(N-Phenyl-2-benzylimidazol)-benzol) (TPBI), wobei TPBI und BAIq auch als Elektronen-leitende Materialien geeignet sind. In einer weiteren Ausführungsform können Verbindungen, die aromatische oder heteroaromatische über Carbonyl-Gruppen enthaltende Gruppen verbundene Ringe enthalten, wie sie in WO2006/100298 offenbart sind, als Blockschicht für Löcher/Excitonen (4) oder als Matrixmaterialien in der Licht-emittierenden Schicht (3) eingesetzt werden.

In verschiedenen Ausführungsformen betrifft die vorliegende Erfindung eine erfindungsgemäße OLED umfassend die Schichten (1) Anode, (2) Lochleiterschicht, (3) Licht-emittierende Schicht, (4) Blockschicht für Löcher/Excitonen, (5) Elektronenleiter- schicht und (6) Kathode, sowie gegebenenfalls weitere Schichten, wobei die Licht-emittierende Schicht (3) mindestens eine Verbindung der Formeln (II), (III) und (IV) enthält.

Geeignete Elektronenleitermaterialien für die Schicht (5) der erfindungsgemäßen OLEDs umfassen mit oxinoiden Verbindungen chelatisierte Metalle wie Tris(8-chinolinolato)aluminium (Alqß), Bis-(2-methyl-8-chinolinato)-4-phenylphenylato)-aluminium(III) (BAIq), Verbindungen auf Phenanthrolinbasis wie 2,9-Dimethyl-4,7-diphenyl-1,10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ) und 2,2',2"-(1,3,5-phenylen)tris-[1-phenyl-1H-benzimidazol] (TPBI). Dabei kann die Schicht (5) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (5) die Beweglichkeit der Elektronen und reduziert ein Quenchen des Excitons. Bevorzugt geeignete Elektronenleitermaterialien sind TPBI und BAIq.

Von den vorstehend als Lochleitermaterialien und Elektronenleitermaterialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen. Diese können z. B. in der Blockschicht für Löcher/Excitonen (4) eingesetzt werden. Es ist jedoch ebenfalls möglich, dass die Funktion als Loch/Excitonenblocker von der Schicht (5) mit übernommen wird, so dass die Schicht (4) entfallen kann.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können die Lochleitermaterialien mit Elektronenakzeptoren dotiert werden, zum Beispiel können Phthalocyanine bzw. Arylamine wie TPD oder TDTA mit Tetrafluortetracyanchinodimethan (F4-TCNQ) dotiert werden. Die Elektronenleitermaterialien können zum Beispiel mit Alkalimetallen dotiert werden, beispielsweise Alq₃ mit Lithium. Die elektronische Dotierung ist dem Fachmann bekannt und zum Beispiel in W. Gao, A. Kahn, J. Appl. Phys., Vol. 94, No. 1 , 1 July 2003 (p-dotierte organische Schichten); A. G. Werner, F. Li, K. Harada, M. Pfeiffer, T. Fritz, K. Leo. Appl. Phys. Lett., Vol. 82, No. 25, 23 June 2003 und Pfeiffer et al., Organic Electronics 2003, 4, 89 - 103 offenbart.

Die Kathode (6) ist eine Elektrode, die zur Einführung von Elektronen oder negativen Ladungsträgern dient. Geeignete Materialien für die Kathode sind ausgewählt aus der Gruppe bestehend aus Alkalimetallen der Gruppe Ia, zum Beispiel Li, Cs, Erdalkalimetallen der Gruppe IIa, zum Beispiel Calcium, Barium oder Magnesium, Metallen der Gruppe IIb des Periodensystems der Elemente (alte ILJPAC-Version), umfassend die Lanthaniden und Aktiniden, zum Beispiel Samarium. Des Weiteren können auch Metalle wie Aluminium oder Indium, sowie Kombinationen aller genannten Metalle eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In verschiedenen Ausführungsformen enthält das erfindungsgemäße OLED zusätzlich zu den Schichten (1) bis (6) mindestens eine der im Folgenden genannten weiteren Schichten:
Eine Loch-Injektionsschicht zwischen der Anode (1) und der Löcher- transportierenden Schicht (2); eine Blockschicht für Elektronen zwischen der Löcher-transportierenden Schicht (2) und der Licht-emittierenden Schicht (3); eine Elektronen-Injektionsschicht zwischen der Elektronentransportierenden Schicht (5) und der Kathode (6).

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z.B. in WO 00/70655 offenbart.

Des Weiteren ist es möglich, dass einige oder alle der in der erfindungsgemäßen OLED eingesetzten Schichten oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz und Lebensdauer zu erhalten.

Die Herstellung des erfindungsgemäßen OLEDs kann nach dem Fachmann bekannten Methoden erfolgen. Im Allgemeinen wird das erfindungsgemäße OLED durch aufeinander folgende Dampfabscheidung (Vapor Deposition) der einzelnen Schichten auf ein geeignetes Substrat hergestellt. Geeignete Substrate sind zum Beispiel Glas, anorganische Halbleiter oder Polymerfilme. Zur Dampfabscheidung können übliche Techniken eingesetzt werden wie thermische Verdampfung, Chemical Vapor Deposition (CVD), Physical Vapor Deposition (PVD) und andere. In einem alternativen Verfahren können die organischen Schichten der OLED aus Lösungen oder Dispersionen in geeigneten Lösungsmitteln aufgetragen werden, wobei dem Fachmann bekannte Beschichtungstechniken angewendet werden.

Im Allgemeinen haben die verschiedenen Schichten folgende Dicken: Anode (1) 50 bis 500 nm, bevorzugt 100 bis 200 nm; Löcher-leitende Schicht (2) 5 bis 100 nm, bevorzugt 20 bis 80 nm, Licht-emittierende Schicht (3) 1 bis 100 nm, bevorzugt 10 bis 80 nm, Blockschicht für Löcher/Excitonen (4) 2 bis 100 nm, bevorzugt 5 bis 50 nm, Elekt- ronen-leitende Schicht (5) 5 bis 100 nm, bevorzugt 20 bis 80 nm, Kathode (6) 20 bis 1000 nm, bevorzugt 30 bis 500 nm. Die relative Lage der Rekombinationszone von Löchern und Elektronen in dem erfindungsgemäßen OLED in Bezug zur Kathode und somit das Emissionsspektrum des OLED können u. a. durch die relative Dicke jeder Schicht beeinflusst werden. Das bedeutet, die Dicke der Elektronentransportschicht sollte bevorzugt so gewählt werden, dass die Lage der Rekombinationszone auf die optische Resonatoreigenschaft der Diode und damit auf die Emissionswellenlänge des Emitters abgestimmt ist. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt. Es ist möglich, dass die Elektronen-leitende Schicht und/oder die Löcher-leitende Schicht größere Dicken als die angegebenen Schichtdicken aufweisen, wenn sie elektrisch dotiert sind.

Erfindungsgemäß enthält die Licht-emittierende Schicht und/oder mindestens eine der weiteren in der erfindungsgemäßen OLED gegebenenfalls vorliegenden Schichten mindestens eine Verbindung der allgemeinen Formeln (II), (III) und (IV). Während die mindestens eine Verbindung der allgemeinen Formel (I) in der Licht-emittierenden Schicht als Emitter- und/oder Matrixmaterial vorliegt, kann die mindestens eine Verbindung der allgemeinen Formeln (II), (III) und (IV) in der mindestens einen weiteren Schicht der erfindungsgemäßen OLED jeweils allein oder gemeinsam mit mindestens einem der weiteren für die entsprechenden Schichten geeigneten vorstehend genannten Materialien eingesetzt werden. Es ist ebenfalls möglich, dass die Licht-Emittierende Schicht neben der Verbindung der Formeln (II), (III) oder (IV) ein oder mehrere weitere Emitter- und/oder Matrixmaterialien enthält.

Die Effizienz der erfindungsgemäßen OLEDs z.B. durch Optimierung der einzelnen Schichten verbessert werden. Beispielsweise können hoch effiziente Kathoden wie Ca oder Ba, gegebenenfalls in Kombination mit einer Zwischenschicht aus LiF, eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, in denen Elektrolumineszenz nützlich ist. Geeignete Vorrichtungen sind bevorzugt ausgewählt aus stationären und mobilen Bildschirmen und Beleuchtungseinheiten. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Digitalkameras, Fahrzeugen sowie Zielanzeigen an Bussen und Bahnen.

Weiterhin können die Verbindungen der Formeln (II), (III) und (IV) in verschiedenen Ausführungsformen in OLEDs mit inverser Struktur eingesetzt werden. Der Aufbau von inversen OLEDs und die üblicherweise darin eingesetzten Materialien sind dem Fachmann bekannt.

Alle zitierten Dokumente sind hierin in ihrer Gesamtheit durch Bezugnahme eingeschlossen. Weitere Ausführungsformen finden sich in den folgenden Beispielen, ohne dass die Erfindung auf diese beschränkt ist.

Es ist selbstverständlich und beabsichtigt, dass alle Ausführungsformen die hierin im Zusammenhang mit den beschriebenen Verbindungen offenbart sind, in gleichem Maße auf die beschriebenen Verwendungen und Verfahren anwendbar sind und umgekehrt. Derartige Ausführungsformen fallen daher ebenfalls in den Umfang der vorliegenden Erfindung.

### Beispiele

### 9-(2,3,5,6-Tetrafluoro-4-(trifluoromethyl)phenyl)-9H-carbazol (4F1Cz)

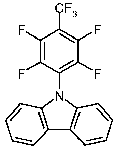

Das Produkt wurde durch Reaktion von Octofluorotoluol (1,41 g; 5,98 mmol) mit Carbazol (1 g; 7,2 mmol) in Anwesenheit von Kaliumcarbonat (0,99 g; 7,2 mmol) in 10 ml trockenem DMF (Dimethylformamid) erhalten. Die Reaktionsmischung wurde über einen Zeitraum von 26 Stunden bei Raumtemperatur gerührt. Die Zielsubstanz wurde säulenchromatographisch aufgereinigt und mit einer Ausbeute von 86 % (1,46 g) erhalten. Die Photolumineszenz in Toluol wurde bestimmt und ist in Abb. 2 gezeigt.

### 9-(2,4,6-Trichloro-3,5-difluorophenyl)-9H-carbazol

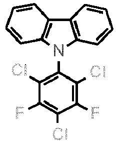

Das Produkt wurde durch Reaktion von 1,3,5-Trichloro-2,4,6-trifluorobenzol (1,98 g; 14,4 mmol) mit Carbazol (2 g; 11,98 mmol) in Anwesenheit von Kaliumcarbonat (3,3 g; 23,9 mmol) in 10 ml trockenem DMSO (Dimethylsulfoxid) erhalten. Die Reaktionsmischung wurde über einen Zeitraum von 7 Stunden und unter Erhitzen auf 50 °C gerührt. Die Zielsubstanz wurde säulenchromatographisch aufgereinigt und mit einer Ausbeute von 13 % (0,56 g) erhalten.

### 4-(9H-Carbazol-9-yl)-3-(trifluoromethyl)benzonitril

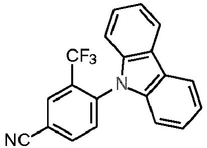

Das Produkt wurde durch Reaktion von 4-Fluoro-3-(trifluoromethyl)benzonitril (1 g; 5,2 mmol) mit Carbazol (0,88 g; 5,2 mmol) in Anwesenheit von Kaliumcarbonat (1,44 g; 10,4 mmol) in 10 ml trockenem DMF (Dimethylformamid) erhalten. Die Reaktionsmischung wurde über einen Zeitraum von 20 Stunden und unter Erhitzen auf 80 °C gerührt. Die Zielsubstanz wurde mittels Umkristallisation aus einer Ether/Hexan-Mischung aufgereinigt und mit einer Ausbeute von 47 % (0,8 g) erhalten.

### 9-(3-Carbazol-9-yl-2,4,6-trichloro-5-fluorophenyl)carbazol

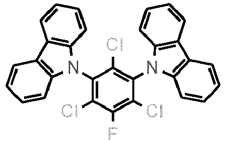

Das Produkt wurde durch Reaktion von 1,3,5-Trichloro-2,4,6-trifluorobenzol (1,98 g; 14,4 mmol) mit Carbazol (2 g; 11,98 mmol) in Anwesenheit von Kaliumcarbonat (3,3 g; 23,9 mmol) in 10 ml trockenem DMSO (Dimethylsulfoxid) erhalten. Die Reaktionsmischung wurde über einen Zeitraum von 7 Stunden und unter Erhitzen auf 50 °C gerührt. Die Zielsubstanz wurde säulenchromatographisch aufgereinigt und mit einer Ausbeute von 52 % (1,63 g) erhalten.

### 9,9'-(2,4,5-Trifluoro-6-(trifluoromethyl)-1,3-phenylen)bis(9H-carbazol) (3F2Cz)

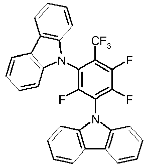

Das Produkt wurde durch Reaktion von Octofluorotoluol (1,5 g; 6,3 mmol) mit Carbazol (2,12 g; 12,7 mmol) in Anwesenheit von Kaliumcarbonat (1,93 g; 13,9 mmol) in 10 ml trockenem DMF (Dimethylformamid) erhalten. Die Reaktionsmischung wurde über einen Zeitraum von 24 Stunden bei Raumtemperatur gerührt. Die Zielsubstanz wurde säulenchromatographisch aufgereinigt und mit einer Ausbeute von 53 % (1,76 g) erhalten. Die Photolumineszenz in Toluol wurde bestimmt und ist in Abb. 2 gezeigt.

### 9,9',9"-(2,4-Difluoro-6-(trifluoromethyl)benzo-1,3,5-triyl)tris(9H-carbazol) (2F3Cz) (nicht erfindungsgemäß)

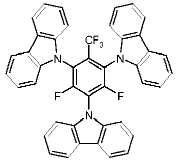

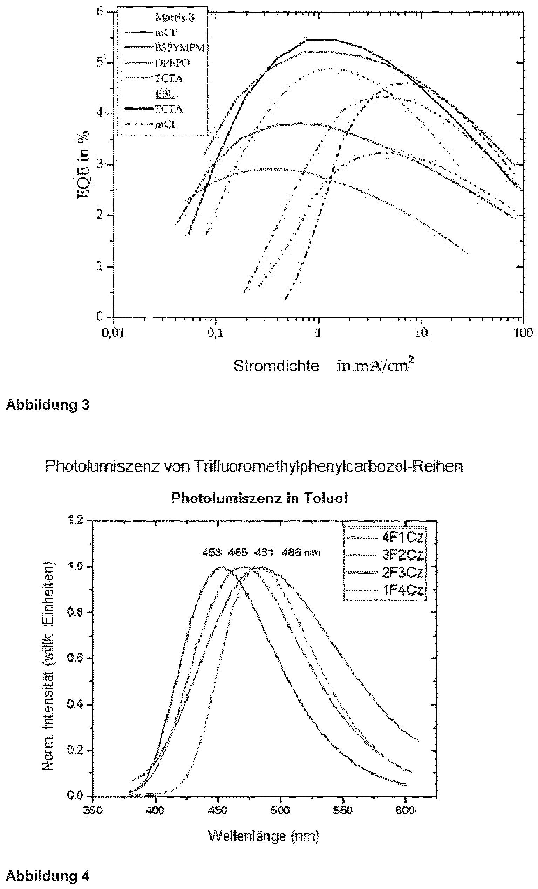

Das Produkt wurde durch Reaktion von Octofluorotoluol (1,5 g; 6,3 mmol) mit Carbazol (2,83 g; 16,9 mmol) in Anwesenheit von Kaliumhydroxid (1,93 g; 33,9 mmol) in 35 ml trockenem Aceton erhalten. Die Reaktionsmischung wurde über einen Zeitraum von einer Stunde zum Reflux erhitzt. Die Zielsubstanz wurde säulenchromatographisch aufgereinigt und mit einer Ausbeute von 53 % (2,42 g) erhalten. Die Photolumineszenz in Toluol wurde bestimmt und ist in Abb. 2 gezeigt. 1F4Cz in Abb. 2 bezeichnet 9,9',9",9"'-(2-Fluoro-6-(trifluoromethyl)benzo-1,3,4,5-tetrayl)tetrakis(9H-carbazol) (1F4Cz). Für die Verbindung 1F4Cz ist in Abb. 3 auch die EQE in % in verschiedenen Hostmaterialien gezeigt B3PYMPM = bis-4,6-(3,5-di-3-pyridylphenyl)-2-methylpyrimidine, mCP = 1,3-Bis-(N-Carbazoyl)benzen, TCTA = Tris(4-Carbazoyl-9-ylphenyl)amin).

## Patentansprüche

1. Verbindung der Formel (II) 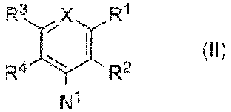 wobei
X C-CF₃ oder C-CN ist;
N¹ bezeichnet einen substituierten oder unsubstituierten Phenoxazin-, Phenothiazin-, Indol-, Benzimidazol-, Chinolin-, Dibenzazepin-, Carbazol- oder 9H-Acridinrest, der jeweils über das Stickstoffatom an den Aromaten der Formel (II) gebunden ist;
R² bezeichnet CF₃ oder F; und
R¹, R³ und R⁴ bezeichnen jeweils unabhängig voneinander F oder H.

2. Verbindung der Formel (III) 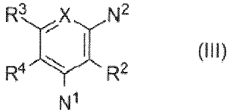 wobei
X C-CF₃, C-SCN, C-CCl₃, C-Cl, C-F, C-CN oder N ist;
N¹ ein Stickstoff-haltiger Heteroarylrest ist, der über ein Stickstoffatom gebunden ist und ausgewählt ist aus den folgenden substituierten oder unsubstituierten Verbindungen: Indolyl, Isoindolyl, Carbazolyl, Piperidinyl, Tetrahydrochinolinyl, Pyrrolidinyl, Tetrahydroisochinolinyl, Acridinyl, Phenanthridinyl, Benzo-5,6-chinolinyl, Benzomorpholinyl, Benzothiomorpholinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, Benzopyridazinyl, Benzpyrimidinyl, Chinoxalinyl, Pyrazinyl, Phenazinyl, Naphthyridinyl, Benzocarbolinyl, Purinyl, Pteridinyl, oder Indolizinyl; und N² unabhängig davon ein Stickstoff-haltiger Heteroarylrest, der über ein Stickstoffatom gebunden ist, oder ein Rest der Forme! -N(Ar)₂, -N(HetAr)(Ar), -N(HetAr)₂ sind, wobei jedes Ar unabhängig ein Arylrest und jedes HetAr unabhängig ein Heteroarylrest ist; und
R², R³ und R⁴ jeweils unabhängig voneinander F, Cl, Br, CCl₃, C1-C12 Perfluoralkyl, insbesondere CF₃, CN oder H sind;
mit der Maßgabe, dass
i. nur einer der Substituenten R^{2,3,4} CN ist;
ii. wenn X C-CN ist, keiner der Substituenten R^{2,3,1} CN ist.

3. Verbindung der Formel (IV) 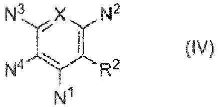 wobei
X C-CF₃, C-Cl, C-F, C-SCN, C-CCl₃, C-CN oder N ist;
R² F, Cl, Br, SCN, CF₃, CN oder H ist; und
N¹ ein Stickstoff-haltiger Heteroarylrest ist, der über ein Stickstoffatom gebunden ist und ausgewählt ist aus den folgenden substituierten oder unsubstituierten Verbindungen: Indolyl, Isoindolyl, Carbazolyl, Piperidinyl, Tetrahydrochinolinyl, Pyrrolidinyl, Tetrahydroisochinolinyl, Acridinyl, Phenanthridinyl, Benzo-5,6-chinolinyl, Benzomorpholinyl, Senzothiomorpholinyl, Benzo-6,7-chinolinyl, Benzo-7,8-chinolinyl, Phenothiazinyl, Phenoxazinyl, Pyrazolyl, Indazolyl, Imidazolyl, Benzimidazolyl, Naphthimidazolyl, Phenanthrimidazolyl, Pyridimidazolyl, Pyrazinimidazolyl, Chinoxalinimidazolyl, Benzopyridazinyl, Benzpyrimidinyl, Chinoxalinyl, Pyrazinyl, Phenazinyl, Naphthyridinyl, Benzocarbolinyl, Purinyl, Pteridinyl, oder Indolizinyl; und N², N³ und N⁴ jeweils unabhängig voneinander ein Stickstoff-haltiger Heteroarylrest, der über ein Stickstoffatom gebunden ist, oder ein Rest der Formel -N(Ar)₂, -N(HetAr)(Ar), -N(HetAr)₂ sind, wobei jedes Ar unabhängig ein Arylrest und jedes HetAr unabhängig ein Heteroarylrest ist; mit der Maßgabe, dass, wenn X C-CN ist, R² nicht CN ist.

4. Verwendung mindestens einer Verbindung gemäß einem der Ansprüche 1 bis 3 in einem optoelektronischen Bauelement, beispielsweise einer organischen Elektrolumineszenzvorrichtung (OLED), einer organischen integrierten Schaltung (O-IC), einem organischen Feld-Effekt-Transistor (O-FET), einem organischen Dünnfilmtransistor (O-TFT), einem organischen lichtemittierenden Transistor (O-LET), einer organischen Solarzelle (O-SC), einem organischen optischen Detektor, einem organischen Photorezeptor, einer organischen Feld-Quench-Vorrichtung (O-FQD), einer lichtemittierenden elektrochemischen Zelle (LEC) oder einer organischen Laserdiode (O-Laser).

5. Optoelektronisches Bauelement, enthaltend mindestens eine Verbindung gemäß einem der Ansprüche 1 bis 3.

## Claims

1. A compound of the formula (II) 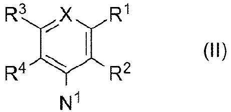 wherein
X is C-CF₃ or C-CN;
N¹ denotes a substituted or unsubstituted phenoxazine, phenothiazine, indole, benzimidazole, quinoline, dibenzazepine, carbazole or 9H-acridine radical, which is bound via the nitrogen atom to the aromatic group of formula (II), respectively; and
R² denotes CF₃ or F; and
R¹, R³, and R⁴ denote each independently F or H.

2. A compound of the formula (III) 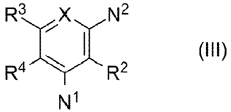 wherein
X is C-CF₃, C-SCN, C-CCl₃, C-Cl, C-F, C-CN or N;
N¹ is a nitrogen-containing heteroaryl radical, which is bound via a nitrogen atom and selected from following substituted or unsubstituted compounds: indolyl, isoindolyl, carbazolyl, piperidinyl, tetrahydroquinolinyl, pyrrolidinyl, tetrahydroisoquinolinyl, acridinyl, phenanthridinyl, benzo-5,6-quinolinyl, benzomorpholinyl, benzothiomorpholinyl, benzo-6,7-quinolinyl, benzo-7,8-quinolinyl, phenothiazinyl, phenoxazinyl, pyrazolyl, indazolyl, imidazolyl, benzimidazolyl, naphthimidazolyl, phenanthrimidazolyl, pyridimidazolyl, pyrazinimidazolyl, quinoxalinimidazolyl, benzopyridazinyl, benzpyrimidinyl, quinoxalinyl, pyrazinyl, phenazinyl, naphthyridinyl, benzocarbolinyl, purinyl, pteridinyl or indolizinyl; and
N² independently thereof is a nitrogen-containing heteroaryl radical bonded via a nitrogen atom, or a radical of the formula -N(Ar)₂, -N(HetAr)(Ar), -N(HetAr)₂, where each Ar is independently an aryl radical and each HetAr is independently a heteroaryl radical; and
R², R³, and R⁴ are each independently F, Cl, Br, CCl₃, C1-C12 perfluoroalkyl, in particular CF₃, CN or H;
with the proviso that
i. only one of the substituents R^{2,3,4} is CN;
ii. when X is C-CN, none of the substituents R^{2,3,4} is CN.

3. A compound of the formula (IV) 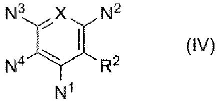 where
X is C-CF₃, C-Cl, C-F, C-SCN, C-CCl₃, C-CN or N;
R² is F, Cl, Br, SCN, CF₃, CN or H; and
N¹ is a nitrogen-containing heteroaryl radical, which is bound via a nitrogen atom and selected from following substituted or unsubstituted compounds: indolyl, isoindolyl, carbazolyl, piperidinyl, tetrahydroquinolinyl, pyrrolidinyl, tetrahydroisoquinolinyl, acridinyl, phenanthridinyl, benzo-5,6-quinolinyl, benzomorpholinyl, benzothiomorpholinyl, benzo-6,7-quinolinyl, benzo-7,8-quinolinyl, phenothiazinyl, phenoxazinyl, pyrazolyl, indazolyl, imidazolyl, benzimidazolyl, naphthimidazolyl, phenanthrimidazolyl, pyridimidazolyl, pyrazinimidazolyl, quinoxalinimidazolyl, benzopyridazinyl, benzpyrimidinyl, quinoxalinyl, pyrazinyl, phenazinyl, naphthyridinyl, benzocarbolinyl, purinyl, pteridinyl, or indolizinyl; and
N², N³, and N⁴ are each independently a nitrogen-containing heteroaryl radical bonded via a nitrogen atom, or a radical of the formula -N(Ar)₂, -N(HetAr)(Ar), -N(HetAr)₂, where each Ar is independently an aryl radical and each HetAr is independently a heteroaryl radical;
with the proviso that
when X is C-CN, R² is not CN.

4. Use of at least one compound according to any of claims 1 to 3 in an optoelectronic component, for example an organic electroluminescent device (OLED), an organic integrated circuit (O-IC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

5. An optoelectronic component comprising at least one compound according to any of claims 1 to 3.

## Revendications

1. Composé de formule (II) 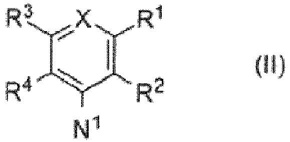 dans laquelle
X représente C-CF₃ ou C-CN ;
N¹ désigne un radical phénoxazine, phénothiazine, indole, benzimidazole, quinoléine, dibenzazépine, carbazole ou 9H-acridine substitué ou non substitué, dont chacun est par l'intermédiaire de l'atome d'azote lié aux composés aromatiques de formule (II) ;
R² désigne CF₃ ou F ; et
R¹, R³ et R⁴ désignent chacun, indépendamment des autres, F ou H.

2. Composé de formule (III) 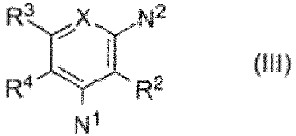 dans laquelle
X représente C-CF₃, C-SCN, C-CCl₃, C-CI, C-F, C-CN ou N ;
N¹ représente un radical hétéroaryle azoté, qui est lié par l'intermédiaire d'un atome d'azote et est choisi parmi les composés substitués ou non substitués suivants : indolyle, isoindolyle, carbazolyle, pipéridinyle, tétrahydroquinoléinyle, pyrrolidinyle, tétrahydroisoquinoléinyle, acridinyle, phénanthridinyle, benzo-5,6-quinoléinyle, benzomorpholinyle, benzothiomorpholinyle, benzo-6,7-quinoléinyle, benzo-7,8-quinoléinyle, phénothiazinyle, phénoxazinyle, pyrazolyle, indazolyle, imidazolyle, benzimidazolyle, naphtimidazolyle, phénanthrimidazolyle, pyridimadazolyle, pyrazinimidazolyle, quinoxalinimidazolyle, benzopyridazinyle, benzopyrimidinyle, quinoxalinyle, pyrazinyle, phénazinyle, naphtypyridinyle, benzocarbolinyle, purinyle, ptéridinyl ou indolizinyle; et
N² représente, indépendamment de ce qui précède, un radical hétéroaryle azoté, qui est lié par l'intermédiaire d'un atome d'azote, ou un radical de formule -N(Ar)₂, - N(HetAr)(Ar), -N(HetAr)₂, chaque Ar représentant d'une manière indépendante un radical aryle et chaque HetAr représentant d'une manière indépendante un radical hétéroaryle ; et
R², R³ et R⁴ représentent chacun indépendamment des autres F, Cl, Br, CCl₃, un radical perfluoralkyle en C₁-C₁₂, en particulier CF₃, CN ou H ;
à la condition que
i. seul un des substituants R^{2,3,4} soit CN ;
ii. quand X représente C-CN, aucun des substituants R^{2,3,4} ne soit CN.

3. Composé de formule (IV) 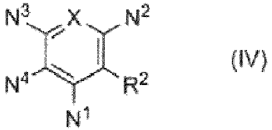 dans laquelle
X représente C-CF₃, C-CI, C-F, C-SCN, C-CCl₃, C-CN ou N ;
R² représente F, Cl, Br, SCN, CF₃, CN ou H ; et
N¹ représente un radical hétéroaryle azoté, qui et lié par l'intermédiaire d'un atome d'azote et est choisi parmi les composés substitués ou non substitués suivants : indolyle, isoindolyle, carbazolyle, pipéridinyle, tétrahydroquinoléinyle, pyrrolidinyle, tétrahydroisoquinoléinyle, acridinyle, phénanthridinyle, benzo-5,6-quinoléinyle, benzomorpholinyle, benzothiomorpholinyle, benzo-6,7-quinoléinyle, benzo-7,8-quinoléinyle, phénothiazinyle, phénoxazinyle, pyrazolyle, indazolyle, imidazolyle, benzimidazolyle, naphtimidazolyle, phénanthrimidazolyle, pyridimadazolyle, pyrazinimidazolyle, quinoxalinimidazolyle, benzopyridazinyle, benzopyrimidinyle, quinoxalinyle, pyrazinyle, phénazinyle, naphtypyridinyle, benzocarbolinyle, purinyle, ptéridinyl ou indolizinyle; et
N², N³ et N⁴ représentent chacun indépendamment des autres un radical hétéroaryle azoté, qui est lié par l'intermédiaire d'un atome d'azote, ou un radical de formule -N(Ar)₂, -N(HetAr)(Ar), -N(HetAr)₂, chaque Ar représentant d'une manière indépendante un radical aryle et chaque HetAr représentant d'une manière indépendante un radical hétéroaryle ;
à la condition que, quand X représente C-CN, R² ne soit pas CN.

4. Utilisation d'au moins un composé selon l'une des revendications 1 à 3 dans un composant optoélectronique, par exemple une diode électroluminescente organique (OLED), un circuit intégré organique (O-IC), un transistor à effet de champ organique (O-FET), un transistor à couche mince organique (O-TFT), un transistor électroluminescent organique (O-LET), une cellule solaire organique (O-SC), un détecteur optique organique, un photorécepteur organique, un dispositif d'extinction de champ organique (O-FQD), une cellule électrochimique électroluminescente (LEC) ou une diode laser organique (O-Laser).

5. Composant optoélectronique contenant au moins un composé selon l'une des revendications 1 à 3.
